# EUROPEAN PATENT SPECIFICATION

(11) **EP 3 076 986 B1**
(45) Date of publication and mention of the grant of the patent: **16.09.2020**
(21) Application number: 14806658.2
(22) Date of filing: 04.12.2014
(51) Int. Cl.: A61K 38/00, A61K 38/55, A61K 39/02, A61K 31/00, A61K 31/11, A61P 3/04

(54) **BACTERIAL INFLUENCE OF HOST FEEDING AND EMOTION VIA CLPB PROTEIN MIMICRY OF A-MSH**
BAKTERIELLER EINFLUSS AUF HOST-FEEDING UND EMOTION MITTELS CLPB-PROTEIN-MIMIKRY EINES A-MSH
INFLUENCE BACTÉRIENNE SUR L'ALIMENTATION ET L'ÉMOTION DE L'HÔTE PAR MIMÉTISME PROTÉIQUE DE LA CLPB AVEC L' A-MSH

(30) Priority: 05.12.2013 EP 13306673
(43) Date of publication of application: 12.10.2016
(73) Proprietor: Institut National de la Santé et de la Recherche Médicale, 75013 Paris (FR); Universite de Rouen, 76130 Mont-Saint-Aignan (FR); Centre Hospitalier Universitaire de Rouen, 76000 Rouen (FR)
(72) Inventor: FETISSOV, Sergueï, F-76183 Rouen (FR); TENNOUNE, Naouel, 60000 Beauvais (FR); CHAN-TCHI-SONG, Philippe, F-76000 Rouen (FR); DE, Emmanuelle, F-76821 Mont-Saint-Aignan Cedex (FR); DECHELOTTE, Pierre, F-76183 Rouen Cedex (FR)
(74) Representative: Icosa
(86) International application number: PCT/EP2014/076654
(87) International publication number: WO 2015/082655

(56) References cited:
- WO-A1-97/03359
- KARIN L. MEIBOM ET AL: "The heat-shock protein ClpB of Francisella tularensis is involved in stress tolerance and is required for multiplication in target organs of infected mice", MOLECULAR MICROBIOLOGY, vol. 67, no. 6, 1 March 2008 (2008-03-01), pages 1384-1401, XP055044187, ISSN: 0950-382X, DOI: 10.1111/j.1365-2958.2008.06139.x
- MARTIN IANIRE ET AL: "Screening and Evaluation of Small Organic Molecules as CIpB Inhibitors and Antimicrobials", JOURNAL OF MEDICINAL CHEMISTRY, AMERICAN CHEMICAL SOCIETY, US, vol. 56, no. 18, 1 September 2013 (2013-09-01), pages 7177-7189, XP009177624, ISSN: 0022-2623
- SINNO M H ET AL: "Regulation of feeding and anxiety by alpha-MSH reactive autoantibodies", PSYCHONEUROENDOCRINOLOGY, OXFORD, GB, vol. 34, no. 1, 1 January 2009 (2009-01-01), pages 140-149, XP025815646, ISSN: 0306-4530, DOI: 10.1016/J.PSYNEUEN.2008.08.021 [retrieved on 2008-12-19]
- HARROLD J A ET AL: "Altered energy balance causes selective changes in melanocortin-4(MC4-R), but not melanocortin-3 (MC3-R), receptors in specific hypothalamic regions: further evidence that activation of MC4-R is a physiological inhibitor of feeding", DIABETES, AMERICAN DIABETES ASSOCIATION, US, vol. 48, no. 2, 1 February 1999 (1999-02-01), pages 267-271, XP002509431, ISSN: 0012-1797, DOI: 10.2337/DIABETES.48.2.267
- MURPHY EILEEN E ET AL: "The Gut Microbiota - A Realistic Therapeutic Target in Obesity and Metabolic Dysregulation", GASTROENTEROLOGY, ELSEVIER, PHILADELPHIA, PA, vol. 140, no. 5, Suppl.1, 1 May 2011 (2011-05-01), pages S103-S104, XP009177630, ISSN: 0016-5085
- BURNET R ET AL: "Neomycin in resistant obesity", ENDOCRINOLOGIA JAPONICA, JAPAN ENDOCRINE SOCIETY, TOKYO, JP, vol. 22, 1 January 1979 (1979-01-01), page 4, XP009108651, ISSN: 0013-7219

### SEQUENCE LISTING

<110> INSERM
   UNIVERSITE ROUEN
   CHU DE ROUEN
<120> Bacterial influence of host feeding and emotion via ClpB protein mimicry of α-MSH
<130> BET14P2920
<150> EP13306673.8
   <151> 2013-12-05
<160> 5
<170> PatentIn version 3.5
<210> 1
   <211> 857
   <212> PRT
   <213> Escherichia coli
<400> 1
   Met Arg Leu Asp Arg Leu Thr Asn Lys Phe Gln Leu Ala Leu Ala Asp
   1 5 10 15
   Ala Gln Ser Leu Ala Leu Gly His Asp Asn Gln Phe Ile Glu Pro Leu
   20 25 30
   His Leu Met Ser Ala Leu Leu Asn Gln Glu Gly Gly Ser Val Ser Pro
   35 40 45
   Leu Leu Thr Ser Ala Gly Ile Asn Ala Gly Gln Leu Arg Thr Asp Ile
   50 55 60
   Asn Gln Ala Leu Asn Arg Leu Pro Gln Val Glu Gly Thr Gly Gly Asp
   65 70 75 80
   Val Gln Pro Ser Gln Asp Leu Val Arg Val Leu Asn Leu Cys Asp Lys
   85 90 95
   Leu Ala Gln Lys Arg Gly Asp Asn Phe Ile Ser Ser Glu Leu Phe Val
   100 105 110
   Leu Ala Ala Leu Glu Ser Arg Gly Thr Leu Ala Asp Ile Leu Lys Ala
   115 120 125
   Ala Gly Ala Thr Thr Ala Asn Ile Thr Gln Ala Ile Glu Gln Met Arg
   130 135 140
   Gly Gly Glu Ser Val Asn Asp Gln Gly Ala Glu Asp Gln Arg Gln Ala
   145 150 155 160
   Leu Lys Lys Tyr Thr Ile Asp Leu Thr Glu Arg Ala Glu Gln Gly Lys
                   165                 170                 175
   Leu Asp Pro Val Ile Gly Arg Asp Glu Glu Ile Arg Arg Thr Ile Gln
               180                 185                 190
   Val Leu Gln Arg Arg Thr Lys Asn Asn Pro Val Leu Ile Gly Glu Pro
           195                 200                 205
   Gly Val Gly Lys Thr Ala Ile Val Glu Gly Leu Ala Gln Arg Ile Ile
       210                 215                 220
   Asn Gly Glu Val Pro Glu Gly Leu Lys Gly Arg Arg Val Leu Ala Leu
   225                 230                 235                 240
   Asp Met Gly Ala Leu Val Ala Gly Ala Lys Tyr Arg Gly Glu Phe Glu
                   245                 250                 255
   Glu Arg Leu Lys Gly Val Leu Asn Asp Leu Ala Lys Gln Glu Gly Asn
               260                 265                 270
   Val Ile Leu Phe Ile Asp Glu Leu His Thr Met Val Gly Ala Gly Lys
           275                 280                 285
   Ala Asp Gly Ala Met Asp Ala Gly Asn Met Leu Lys Pro Ala Leu Ala
       290                 295                 300
   Arg Gly Glu Leu His Cys Val Gly Ala Thr Thr Leu Asp Glu Tyr Arg
   305                 310                 315                 320
   Gln Tyr Ile Glu Lys Asp Ala Ala Leu Glu Arg Arg Phe Gln Lys Val
                   325                 330                 335
   Phe Val Ala Glu Pro Ser Val Glu Asp Thr Ile Ala Ile Leu Arg Gly
               340                 345                 350
   Leu Lys Glu Arg Tyr Glu Leu His His His Val Gln Ile Thr Asp Pro
           355                 360                 365
   Ala Ile Val Ala Ala Ala Thr Leu Ser His Arg Tyr Ile Ala Asp Arg
       370                 375                 380
   Gln Leu Pro Asp Lys Ala Ile Asp Leu Ile Asp Glu Ala Ala Ser Ser
   385                 390                 395                 400
   Ile Arg Met Gln Ile Asp Ser Lys Pro Glu Glu Leu Asp Arg Leu Asp
   405 410 415
   Arg Arg Ile Ile Gln Leu Lys Leu Glu Gln Gln Ala Leu Met Lys Glu
   420 425 430
   Ser Asp Glu Ala Ser Lys Lys Arg Leu Asp Met Leu Asn Glu Glu Leu
   435 440 445
   Ser Asp Lys Glu Arg Gln Tyr Ser Glu Leu Glu Glu Glu Trp Lys Ala
   450 455 460
   Glu Lys Ala Ser Leu Ser Gly Thr Gln Thr Ile Lys Ala Glu Leu Glu
   465 470 475 480
   Gln Ala Lys Ile Ala Ile Glu Gln Ala Arg Arg Val Gly Asp Leu Ala
   485 490 495
   Arg Met Ser Glu Leu Gln Tyr Gly Lys Ile Pro Glu Leu Glu Lys Gln
   500 505 510
   Leu Glu Ala Ala Thr Gln Leu Glu Gly Lys Thr Met Arg Leu Leu Arg
   515 520 525
   Asn Lys Val Thr Asp Ala Glu Ile Ala Glu Val Leu Ala Arg Trp Thr
   530 535 540
   Gly Ile Pro Val Ser Arg Met Met Glu Ser Glu Arg Glu Lys Leu Leu
   545 550 555 560
   Arg Met Glu Gln Glu Leu His His Arg Val Ile Gly Gln Asn Glu Ala
   565 570 575
   Val Asp Ala Val Ser Asn Ala Ile Arg Arg Ser Arg Ala Gly Leu Ala
   580 585 590
   Asp Pro Asn Arg Pro Ile Gly Ser Phe Leu Phe Leu Gly Pro Thr Gly
   595 600 605
   Val Gly Lys Thr Glu Leu Cys Lys Ala Leu Ala Asn Phe Met Phe Asp
   610 615 620
   Ser Asp Glu Ala Met Val Arg Ile Asp Met Ser Glu Phe Met Glu Lys
   625 630 635 640
   His Ser Val Ser Arg Leu Val Gly Ala Pro Pro Gly Tyr Val Gly Tyr
   645 650 655
   Glu Glu Gly Gly Tyr Leu Thr Glu Ala Val Arg Arg Arg Pro Tyr Ser
               660                 665                 670
   Val Ile Leu Leu Asp Glu Val Glu Lys Ala His Pro Asp Val Phe Asn
           675                 680                 685
   Ile Leu Leu Gln Val Leu Asp Asp Gly Arg Leu Thr Asp Gly Gln Gly
       690                 695                 700
   Arg Thr Val Asp Phe Arg Asn Thr Val Val Ile Met Thr Ser Asn Leu
   705                 710                 715                 720
   Gly Ser Asp Leu Ile Gln Glu Arg Phe Gly Glu Leu Asp Tyr Ala His
                   725                 730                 735
   Met Lys Glu Leu Val Leu Gly Val Val Ser His Asn Phe Arg Pro Glu
               740                 745                 750
   Phe Ile Asn Arg Ile Asp Glu Val Val Val Phe His Pro Leu Gly Glu
           755                 760                 765
   Gln His Ile Ala Ser Ile Ala Gln Ile Gln Leu Lys Arg Leu Tyr Lys
       770                 775                 780
   Arg Leu Glu Glu Arg Gly Tyr Glu Ile His Ile Ser Asp Glu Ala Leu
   785                 790                 795                 800
   Lys Leu Leu Ser Glu Asn Gly Tyr Asp Pro Val Tyr Gly Ala Arg Pro
                   805                 810                 815
   Leu Lys Arg Ala Ile Gln Gln Gln Ile Glu Asn Pro Leu Ala Gln Gln
               820                 825                 830
   Ile Leu Ser Gly Glu Leu Val Pro Gly Lys Val Ile Arg Leu Glu Val
           835                 840                 845
   Asn Glu Asp Arg Ile Val Ala Val Gln
       850                 855
<210> 2
   <211> 7
   <212> PRT
   <213> Escherichia coli
<400> 2
   Arg Trp Thr Gly Ile Pro Val
   1               5
<210> 3
   <211> 13
   <212> PRT
   <213> Homo sapiens
<400> 3
   Ser Tyr Ser Met Glu His Phe Arg Trp Gly Lys Pro Val
   1               5                   10
<210> 4
   <211> 21
   <212> DNA
   <213> Artificial Sequence
<220>
   <223> Primer Sequence K12-R
<400> 4
   atcctgcgca ccaatcaaca a 21
<210> 5
   <211> 21
   <212> DNA
   <213> Artificial Sequence
<220>
   <223> Primer sequence
<400> 5
   cgcgatggaa gatgctctgt a 21

## Description

The present invention relates to bacterial ClpB protein and ClpB expressing bacteria and their impact on diseases or disorders related to reduced appetite, increased appetite and/or anxiety. The invention further relates to compositions comprising bacterial ClpB protein and its use in the immunization against a disorder related to reduced appetite and/or anxiety.

Eating disorders increased all over the world among both men and women over the last 50 years. There is evidence to suggest that in particular individuals in the western world are at the highest risk of developing them and the degree of westernization increases the risk. With recent advances, the scientists understand more and more the central processes of appetite. It is known that interactions between motivational, homeostatic and self-regulatory control processes are involved in eating behavior, which is a key component in eating disorders.

Recent science discovered a further regulator, the human microbiome. Advances of high-throughput DNA sequencing technologies allowed to explore the human microbiome and thus to make a major step towards the understanding of the molecular relationships between the host and its microbiota. This "second genome", the microbiome, has been described in a catalogue of more than 4-5 million, non-redundant microbial putative genes and 1 to 2,000 prevalent bacterial species. Each individual has at least 160 shared species and a number of well-balanced host-microbial molecular relationships that defines groups of individuals.

It is considered that understanding the essential features of symbiotic relationships between microbial communities and their human host may allow to predict host phenotypes, such as health status, from the particular features of indigenous communities.

The composition of gut microbiota for example has been associated with host metabolic phenotypes including obesity and diabetes as well as some neuropsychiatric disorders suggesting that gut bacteria may influence brain-controlled functions and behavior.

In this context, determining the molecular mechanisms linking the microbiota to the host behavior appears, thus, as a necessary step for defining the role of specific microorganisms in host physiology.

Recent research of the inventors revealed a new molecular mechanism via molecular mimicry between bacterial proteins and peptide hormones known for their role in regulation of motivated behavior and emotion. The new molecular mechanism via molecular mimicry thus links gut microbiota to host behavior and allowed to identify a new potential therapeutic target in eating disorders and obesity.

The international patent application WO97/03359 discloses *Helicobacter* ClpB polypeptides, immunogenic fragments thereof, or anti *Helicobacter* ClpB antibodies for use as a vaccine against Helicobacter infections. Furthermore, Meibom et al. show that mice immunized with a mutated *Francisella. tularensis* strain expressing a truncated ClpB protein were protected against wild-type *F. tularensis* infection (Meibom et al. Molecular microbiology, 2008, vol. 67, no. 6, pages 1384-1401)

The inventors discovered, using proteomics, that the ClpB chaperon protein of commensal gut bacteria *E. Coli* K12 is a conformational mimetic of α-melanocyte-stimulating hormone (a-MSH), a neuropeptide involved in the regulation of energy metabolism and emotion.

They revealed furthermore that ClpB immunized mice display an increased production of both anti-ClpB and anti-α-MSH antibodies accompanied by increased food intake and body weight and reduced anxiety. Furthermore, providing *E.coli* K12 by gavage in rats also increased plasma levels of ClpB- and α-MSH-reactive immunoglobulins.

Therefore, the presence of ClpB and/or anti-ClpB antibodies may be related to a dysregulation of appetite.

Therefore, the invention also relates to a composition comprising a bacterial ClpB protein for use as a vaccine or as an immunogenic composition.

In particular, said composition is for use in the immunization against a disease or disorder related to reduced appetite and/or anxiety.

More particularly, said composition is for use in the treatment and/or prevention of a disease or disorder related to reduced appetite and/or anxiety.

The disease or disorder related to reduced appetite may be selected from the group consisting of anorexia nervosa (AN), bulimia nervosa (BN), cachexia and wasting diseases.

The present invention also relates to a non-therapeutic method of increasing appetite in a subject, comprising administering to said subject an effective amount of a composition comprising an anti-ClpB antibody.

### Detailed description of the invention

### ClpB expressing bacterium

As used herein, *"ClpB* expressing bacterium" refers to bacteria expressing the chaperone protein ClpB.

The "protein disaggregation chaperone", "chaperone protein ClpB", "ClpB protein" or "ClpB" also known as heat shock protein F84.1 is a member of the Hsp100/ClpB family of hexameric AAA+-ATPases. This family comprises bacterial, fungal, and plant Hsp100 ATPases, ClpB being the bacterial representative. As a part of a stress-induced multi-chaperone system, it is involved in the recovery of the cell from heat-induced damage, in cooperation with the Hsp70 system (DnaK, DnaJ and GrpE) in protein disaggregation, a crucial process for cell survival under stress conditions.

During the infection process, bacterial pathogens encounter stress conditions generated by the host defense to eliminate them and respond to this host defense by increasing the synthesis of heat shock and other stress proteins. In this context, ClpB has been described as an essential factor for acquired thermotolerance and for the virulence and infectivity of several Gram-negative and Gram-positive pathogenic bacteria, such as *Staphylococcus aureus, Francisella turalensis, Listeria monocytogenes, Yersinia enterocolitica, and Salmonella thyphimurium.*

In *E.coli* K12 the chaperone protein ClpB also known as heat shock protein F84.1 or htpM and is a protein of 857 amino acids.

Typically, the chaperone protein ClpB comprises or consists of the amino acid sequence of the chaperone protein ClpB from *E. Coli* K12 with SEQ ID NO: 1 (NCBI Reference Number: NP_417083.1, as available on November 6, 2013 and/or UniProtKB/Swiss-Prot Number: P63284, as available on November 6, 2013). Preferably, the amino acid sequence of chaperone protein ClpB comprises or consists of an amino acid sequence 80 to 100% identical to the amino acid sequence of SEQ ID NO: 1. Preferably, the amino acid sequence is 90 to 100% identical, more preferably 95 to 100%, most preferably 95, 96, 97, 98, 99 or 100% identical to the amino acid sequence of SEQ ID NO: 1.

Thus, a ClpB expressing bacterium refers to a bacterium expressing or overexpressing the chaperone protein ClpB as defined above or a polypeptide comprising or consisting of an amino acid sequence 80 to 100% identical to the amino acid sequence of SEQ ID NO: 1, more preferably 95 to 100%, most preferably 95, 96, 97, 98, 99 or 100% identical to the amino acid sequence of SEQ ID NO: 1.

By a polypeptide having an amino acid sequence at least, for example, 95% "identical" to a query amino acid sequence of the present invention, it is intended that the amino acid sequence of the subject polypeptide is identical to the query sequence except that the subject polypeptide sequence may include up to five amino acid alterations per each 100 amino acids of the query amino acid sequence. In other words, to obtain a polypeptide having an amino acid sequence at least 95% identical to a query amino acid sequence, up to 5% (5 of 100) of the amino acid residues in the subject sequence may be inserted, deleted, or substituted with another amino acid.

In the context of the present application, the percentage of identity is calculated using a global alignment (*i.e.* the two sequences are compared over their entire length). Methods for comparing the identity of two or more sequences are well known in the art. The « needle » program, which uses the Needleman-Wunsch global alignment algorithm (Needleman and Wunsch, 1970 J. Mol. Biol. 48:443-453) to find the optimum alignment (including gaps) of two sequences when considering their entire length, may for example be used. The needle program is, for example, available on the ebi.ac.uk world wide web site. The percentage of identity in accordance with the invention is preferably calculated using the EMBOSS:: needle (global) program with a "Gap Open" parameter equal to 10.0, a "Gap Extend" parameter equal to 0.5, and a Blosum62 matrix.

Proteins consisting of an amino acid sequence "at least 80%, 85%, 90%, 95%, 96%, 97%, 98% or 99% identical" to a reference sequence may comprise mutations such as deletions, insertions and/or substitutions compared to the reference sequence. In case of substitutions, the protein consisting of an amino acid sequence at least 80%, 85%, 90%, 95%, 96%, 97%, 98% or 99% identical to a reference sequence may correspond to a homologous sequence derived from another species than the reference sequence.

Amino acid substitutions may be conservative or non-conservative. Preferably, substitutions are conservative substitutions, in which one amino acid is substituted for another amino acid with similar structural and/or chemical properties. The substitution preferably corresponds to a conservative substitution as indicated in the table below.

| **Conservative substitutions** | **Type of Amino Acid** |
|---|---|
| Ala, Val, Leu, Ile, Met, Pro, Phe, Trp | Amino acids with aliphatic hydrophobic side chains |
| Ser, Tyr, Asn, Gln, Cys | Amino acids with uncharged but polar side chains |
| Asp, Glu | Amino acids with acidic side chains |
| Lys, Arg, His | Amino acids with basic side chains |
| Gly | Neutral side chain |

ClpB expressing bacteria are well-known from the skilled person and may be identified by any conventional technique.

In one embodiment, the ClpB expressing bacterium is selected from the group constituted of the genus *Staphylococcus aureus, Francisella turalensis, Listeria monocytogenes, Yersinia enterocolitica, Salmonella thyphimurium, Escherichia Coli, Enterobacteriaceae, Shigella sonnei, Shigella flexneri, Shigella dysenteriae, Shigella boydii, Citrobacter youngae, Salmonella bongori* and *Salmonella enterica.*

The ClpB protein comprises two nucleotide binding domains (ATP1 and ATP2) and two oligomerization domains (NBD1 and NBD2). The N-terminal domain might function as a substrate-discriminating domain, recruiting aggregated proteins to the ClpB hexamer and/or stabilizing bound proteins. The NBD2 domain is responsible for oligomerization, whereas the NBD1 domain stabilizes the hexamer probably in an ATP-dependent manner. The movement of the coiled-coil domain is essential for ClpB ability to rescue proteins from an aggregated state, probably by pulling apart large aggregated proteins, which are bound between the coiled-coils motifs of adjacent ClpB subunits in the functional hexamer.

The inventors identified that the ClpB chaperon protein of commensal gut bacteria *E.coli* K12 is a conformational mimetic of α-melanocyte-stimulating hormone (a-MSH), a neuropeptide involved in the regulation of energy metabolism and emotion.

"α-MSH", also known as "α-Melanocyte-stimulating hormone", "alpha-MSH", "α-MSH", alpha-melanotropin, alpha-melanocortin, or alpha-intermedin, is a naturally occurring endogenous peptide hormone of the melanocortin family, with a tridecapeptide structure. The amino acid sequence of α-MSH preferably comprises or consists of the amino acid sequence SYSMEHFRWGKPV (SEQ ID NO: 3) (Gen Pept Sequence ID, PRF: 223274, as available on December 2, 2013). However, there exist three types of alpha-melanocyte-stimulating hormone that differ in their acetyl status, the desacetyl alpha MSH, which lacks an acetyl group; mono-acetyl alpha MSH, in which the amino group of the Ser-1 of SEQ ID NO: 3 is acetylated; and di-acetyl alpha MSH, in which both amino and hydroxy groups of the Ser-1 of SEQ ID NO: 3 are acetylated. α-MSH as used herein refers to desacetyl alpha MSH and/or the mono-acetylated and/or the di-acetylated form, in particular to the mono-acetylated α-MSH.

It is critically involved in the regulation of energy balance by decreasing food intake and increasing energy expenditure via activation of the melanocortin receptors type 4 (MC4R), acting both centrally and peripherally. Indeed, in both humans and mice mutations of genes responsible for α-MSH production and for MC4R expression lead to overeating and obesity, while plasma α-MSH levels are associated with body weight changes. Furthermore, α-MSH regulates mood and emotion by increasing anxiety.

"Mimetic" refers to a protein that imitates another protein. This imitation is possible since the protein shares certain characteristics with the protein it mimetics.

A "conformational mimetic" refers to a protein, that shares at least in part the same conformation as another protein.

The inventors demonstrated that the ClpB protein shares a discontinuous sequence homology between amino acids 542 to 548 from SEQ ID NO: 1 with α-MSH, of amino acid sequence 'RWTGIPV' (referenced under SEQ ID NO: 2). Without being bound by theory, this conformation of ClpB allows stimulating the production of antibodies directed against both ClpB and α-MSH.

Thus "conformational mimetic" herein preferably refers to the capability to stimulate antibody production against ClpB as well as auto antibodies against α-MSH.

### Subject

In the context of the present invention, a "subject" denotes a human or non-human mammal, such as a rodent (rat, mouse, rabbit), a primate (chimpanzee), a feline (cat), or a canine (dog). Preferably, the subject is human. The subject according to the invention may be in particular a male or a female.

Preferably, the subject according to the invention is older than 13.

Preferably, the subject is a female.

The subject preferably suffers from dysregulated appetite.

By "appetite" is meant the desire to eat food, felt as hunger. Appetite exists in all higher life-forms, and serves to regulate adequate energy intake to maintain metabolic needs. It is regulated by a close interplay between the digestive tract, adipose tissue and the brain. Appetite is assessed in a subject by measuring the amount of food ingested and by assessing the subject's desire to eat.

"Dysregulation of appetite" refers to an abnormal appetite which includes increased appetite as well as decreased appetite which is permanently present or reoccurs several times a week and thus contributes to anorexia nervosa, bulimia nervosa, cachexia, wasting disease, overeating, binge eating disorder and hyperphagia.

"Increased appetite" refers to an appetite wherein a subject has a higher food intake than the body requires. This may or may not result in weight gain.

"Normal appetite" thus refers to a subject having a food intake that corresponds to the amount of food the body requires.

"Decreased appetite" and/or "reduced appetite" refers an appetite wherein a subject has a lower food intake than the body requires. This may or may not result in weight loss.

In one particular embodiment, the subject suffers from overweight.

In the same embodiment, the subject may suffer from increased appetite.

"Overweight subject" refers herein to a subject preferably having a BMI of 25 to 30.

In one embodiment, the subject suffers from obesity.

"Obesity" refers herein to a medical condition wherein the subject preferably has a BMI of >30.

In one embodiment, the subject suffering from overweight and/or obesity might suffer from binge eating disorder and/or hyperphagia. The "BMI" or "body mass index" is defined as the subject's body mass divided by the square of his height. The formulae universally used in medicine produce a unit of measure of kg/m².

"Binge eating disorder" or "BED" refers to an eating disorder characterized by binge eating consisting of eating, in a discrete period of time (e.g., within any 2-hour period), an amount of food that is larger than most people would eat in a similar period of time under similar circumstances, and is accompanied by a feeling of loss of control. The binge eating occurs, on average, at least twice a week for 6 months. Contrary to bulimia the binge eating is not associated with the recurrent use of inappropriate compensatory behavior. Subjects suffering from BED are seriously worried about the binge eating. Furthermore, subjects suffering from BED eat until being physically uncomfortable and nauseated due to the amount of food consumed and/or eat when bored or depressed and/or eat large amounts of food even when not really hungry and/or eat alone during periods of normal eating, owing to feelings of embarrassment about food and/or feel disgusted, depressed, or guilty after binge eating. Subjects with binge eating disorder act impulsively and feel a lack of control over their eating. Furthermore, subject suffering from binge eating disorder have problems coping with stress, anxiety, anger, sadness, boredom and worry.

In the context of this invention, subjects suffering of BED are preferably obese and have a BMI of >30.

"Hyperphagia", also known as "polyphagia" refers to excessive hunger (compulsive) or increased appetite. In one embodiment hyperphagia may be caused by disorders such as Diabetes, Kleine-Levin Syndrome, the genetic disorders Prader-Willi Syndrome and Bardet Biedl Syndrome.

In one embodiment, the subject suffers from reduced appetite and/or anxiety.

In another embodiment, the subject suffers from weight loss.

The subject may further suffer from a disease or disorder related to reduced appetite and/or anxiety.

In one embodiment, said subject may suffer from reduced appetite and/or loss of appetite.

"Anxiety" and/or "Anxiety Disorders" or "disease or disorder related to anxiety" as used herein refers to a large number of disorders where the primary feature is abnormal or inappropriate anxiety.

In one embodiment anxiety and/or anxiety disorder relates to inappropriate anxiety. Inappropriate anxiety refers to the appearance of anxiety symptoms such as increased heart rate, tensed muscles, increased breathing without any recognizable stimulus or a stimulus that does not warrant such a reaction. In one embodiment, these symptoms are related to anxious-avoidant, obsessive-compulsive and perfectionist types of temperament. Once a medical cause is ruled out, an anxiety disorder may be the reason.

In one embodiment, the disease or disorder related to anxiety is selected from the group consisting of acute stress disorder, agoraphobia (with or without a history of panic disorder), generalized anxiety disorder [GAD], obsessive-compulsive disorder [OCD], panic disorder (with or without agoraphobia), phobias (including social phobia) and posttraumatic stress disorder (PTSD).

In another embodiment anxiety may further relate to inappropriate anxiety associated with anorexia nervosa, bulimia nervosa, cachexia, or wasting disease, in particular anorexia nervosa or bulimia nervosa.

Therefore, anxiety may relate for example to the irrational fear of gaining weight and/or the irrational fear of being not perfect.

"Disease or disorder related to reduced appetite" refers in general to diseases or disorders wherein the primary feature is reduced food intake and/or weight loss. The term "disease or disorder related to reduced appetite" refers herein in particular to diseases and/or disorders such as anorexia nervosa, bulimia nervosa, cachexia, or wasting disease, in particular anorexia nervosa and/or bulimia nervosa.

"Anorexia" relates to the decreased sensation of appetite thus resulting in a reduced appetite. While the term in non-scientific publications is often used interchangeably with anorexia nervosa, many possible causes exist for a decreased appetite, some of which may be harmless, while others indicate a serious clinical condition or pose a significant risk.

"Anorexia nervosa" (AN) refers in the context of the invention to an eating disorder characterized by immoderate food restriction that is characterized by failure to maintain body weight of at least 85% of the normal body weight. Furthermore the subject suffering from anorexia nervosa has an irrational fear of gaining weight, as well as a distorted body self-perception, where the subject sees him/herself as overweight despite overwhelming evidence to the contrary.

"Normal body weight" refers herein to body weight resulting in a BMI of between 18.5 and 25.

Accordingly, a person suffering from anorexia nervosa preferably disposes at least one of the psychological traits selected from the group consisting of body dissatisfaction, drive for thinness, perfectionism, ineffectiveness, interpersonal distrust, social insecurity and anhedonia. Preferably, a person suffering from anorexia nervosa disposes at least one of the psychological traits selected from the group consisting of body dissatisfaction, drive for thinness and perfectionism. Still preferably, a person suffering from anorexia nervosa disposes at least one of the psychological traits selected from the group consisting of ineffectiveness, interpersonal distrust, social insecurity and anhedonia.

In a further embodiment, a subject suffering from anorexia nervosa has a BMI of < 17.

"Bulimia" or "Bulimia nervosa" is an eating disorder characterized by binge eating and purging, or consuming a large amount of food in a short amount of time followed by an attempt to rid oneself of the food consumed (purging), typically by vomiting, taking a laxative or diuretic, and/or excessive exercise. Some subjects may tend to alternate between bulimia nervosa and anorexia nervosa. Subject suffering from bulimia may be characterized by a normal BMI range, usually < 25.

"Cachexia" is a wasting syndrome associated with loss of weight and/or muscle atrophy and/or fatigue, weakness, and significant loss of appetite which may be caused by cancer, AIDS, chronic obstructive lung disease, multiple sclerosis, congestive heart failure, tuberculosis, familial amyloid polyneuropathy, mercury poisoning (acrodynia) and hormonal deficiency. Cachexia, or wasting, as it may also be called, is seen with several diseases, such as AIDS, cancer, post hip fracture, chronic heart failure, chronic lung disease such as chronic obstructive lung disease (COLD) and/or Chronic obstructive pulmonary disease (COPD), liver cirrhosis, renal failure, and autoimmune diseases such as rheumatoid arthritis and systemic lupus, sepsis and severe infection. Furthermore, wasting is also seen in aging.

In some embodiments, cachexia is caused by cancer.

The foremost sign of cachexia is weight loss, not only of fatty tissue but also of muscle tissue and even bone. This non-fatty tissue is also known as "lean body mass."

In addition, there is loss of appetite, weakness (asthenia), and a drop in hemoglobin level (anemia).

Cachexia is found as the terminal state of many different clinical conditions or in chronic diseases such as cancer, infections, AIDS, congestive heart failure, rheumatoid arthritis, tuberculosis, post-hip fracture, cystic fibrosis and Crohn's disease. It can also occur in elderly people who do not have any obvious symptoms of disease.

In one embodiment, the subject according to the invention does not have any infection and is free of bacteremia. Accordingly, the subject according to the invention preferably displays a plasma fibrinogen concentration lower than 10 mg/l, in particular lower than 5 mg/l, and/or does not present an abundant leukocyturia and/or does not take antiviral therapy and/or displays a plasma baseline C reactive protein concentration lower than 30 mg/l.

As used herein, the term "C reactive protein" or "CRP" refers to a protein which is a member of the class of acute-phase reactants, as its levels rise dramatically during inflammatory processes occurring in the body. As known from the skilled person, CRP is a 224-residue protein with a monomer molar mass of 25106 Da, encoded by the *CRP* gene.

As used herein, the term "fibrinogen" refers to a 340000 Da plasma glycoprotein which is cleaved to fibrin by thrombin during the final stages of the blood coagulation cascade.

As used herein, the term "leukocyturia" refers to the presence of leukocytes in the urine of the subject. In particular, an abundant leukocyturia corresponds to the presence of 100,000 leukocytes per minute in the urine.

The inventors discovered, using proteomics, that the ClpB chaperon protein of commensal gut bacteria *E.Coli* K12 is a conformational mimetic of α-melanocyte-stimulating hormone (a-MSH), a neuropeptide involved in the regulation of energy metabolism and emotion.

The inventors further discovered that ClpB immunized mice have higher plasma levels of anti-ClpB IgG and increased plasma levels of anti-α-MSH-reactive IgG.

Furthermore, ClpB immunized mice had an increased appetite contributing to overeating and resulting in higher body weight.

In this context, the inventors showed that rats that were gavaged with *E.Coli* K12 expressing ClpB have increased amounts of anti-ClpB IgG and anti-α-MSH IgM.

Thus, the presence of ClpB expressing bacteria results in increased appetite contributing to overeating.

In the context of the invention, the term "treating" or "treatment", as used herein, means reversing, alleviating, inhibiting the progress of the disorder or condition to which such term applies, or one or more symptoms of such disorder or condition.

"Preventing" refers to measures taken to prevent the disorder or condition to which such term applies from occurrence or, in early stages of a disease or disorder. Preventing further refers to the inhibition of further development of a disorder or condition to which such term applies.

The inventors discovered that increased appetite is associated with the presence of ClpB protein and/or anti-ClpB antibodies and increased plasma levels of anti-α-MSH-reactive antibodies, preferably anti-ClpB IgG and/or IgM and anti-α-MSH-reactive IgG and/or IgM.

In one embodiment, reducing the amount or concentration of ClpB expressing bacteria in a subject results in the reduction and/or normalization of appetite in said subject.

Without being bound by theory, the composition comprising at least one antibiotic directed against at least one ClpB expressing bacterium reduces the amount of ClpB expressing bacteria, thus reducing the level or concentration of anti-ClpB antibodies and/or reducing the level of anti-α-MSH-reactive antibodies, thus resulting in a normalized and/or reduced appetite.

"Appetite", "increased appetite" and "normalized appetite" have been defined in the section *"Subject"* above.

"Reduced appetite" refers herein to a normalized appetite that is reduced in comparison to the previously increased appetite.

"Food intake" can be measured using a multitude of techniques including self-reporting using e.g. diaries or questionnaires, measurements of calorie-intake from a buffet meal, using weighing of food prior to ingestion, or weighing and analysis of paired quantities of food. The food intake may be measured on a meal basis, a daily basis, a weekly basis or a monthly basis.

ClpB expressing bacterium has been defined in the section *"ClpB expressing bacterium"* above.

### Treatment and prevention of a disease or disorder related to reduced appetite and/or anxiety

The inventors discovered that ClpB immunized mice have higher plasma levels of anti-ClpB antibodies and surprisingly increased plasma levels of anti-α-MSH auto-antibodies, in particular anti-ClpB IgG cross-reacting with α-MSH.The inventors further discovered that ClpB immunized mice had a better protection against α-MSH anorexigenic effects and a reduction of α-MSH-mediated anxiety.

Accordingly, the invention refers to a composition comprising a bacterial ClpB protein for use as a vaccine or as an immunogenic composition.

By "immunogenic composition" is meant in the context of the present invention a composition comprising a bacterial ClpBprotein which provokes or immunomodulates *(i.e.* immunosuppress or immunostimulate) an immune response when administered to an individual or which induces the production of antibodies when administered to an individual.

By "vaccine" is meant a composition, such as the immunogenic composition described herein which is administered to immunomodulate an immune response, that will protect or treat an individual from illness, in particular due to that agent. The vaccine of the present invention is intended for use both as a therapeutic (treatment) vaccine, *i.e.* for administration to the individual after development of the disease with the intention to reduce or arrest disease progression and as a preventive (prophylactic) vaccine, for administration to the individual prior to the development of the disease.

In particular, said composition is for use in the immunization against a disease or disorder related to reduced appetite and/or anxiety.

More particularly, said composition is for use in the treatment and/or prevention of a disease or disorder related to reduced appetite and/or anxiety.

Without being bound by theory, in one embodiment, the composition comprising a bacterial ClpBprotein can increase the plasma levels of anti-ClpB antibodies and/or plasma levels of anti-α-MSH-reactive antibodies, in particular the plasma levels of anti-ClpBIgG and/or plasma levels of anti-α-MSH-reactive IgG.

Derivatives, fragments or variants of ClpB retaining the ability to increase plasma levels of anti-ClpB antibodies and/or plasma levels of anti-α-MSH-reactive antibodies are also within the scope of the invention.

"Variants" of ClpB protein may be naturally occurring variants, such as splice variants, alleles and isoforms, or they may be produced by recombinant means. Variations in amino acid sequence may be introduced by substitution, deletion or insertion of one or more codons into the nucleic acid sequence encoding the protein that results in a change in the amino acid sequence of the protein. Optionally the variation is by substitution of 1, 2, 3, 4, 5, 6, 7, 8, 9, 10, 11, 12, 13, 14, 15, 16, 17, 18, 19, 20 or more amino acids with any other amino acid in the protein. Additionally or alternatively, the variation may be by addition or deletion of 1, 2, 3, 4, 5, 6, 7, 8, 9, 10, 11, 12, 13, 14, 15, 16, 17, 18, 19, 20, 40 or 50 or more amino acids within the protein.

"Fragments" of the ClpB protein may be truncated at the N-terminus or C-terminus, or may lack internal residues, for example, when compared with a full length protein. Certain fragments lack amino acid residues that are not essential for enzymatic activity. Preferably, said fragments are at least about 10, 20, 30, 40, 50, 60, 70 or more amino acids shorter than the ClpB protein. Furthermore, "derivatives" comprise C- or N-terminally tagged fragments or variants of ClpB protein.

ClpB protein variants may include proteins that have at least about 80% amino acid sequence identity with a polypeptide sequence disclosed herein. Preferably, a variant protein will have at least about 50%, 55%, 60%, 65%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% amino acid sequence identity to a full-length polypeptide sequence or a fragment of a polypeptide sequence as disclosed herein.

"Identity" is defined in the section "*ClpB expressing bacterium"* above.

The inventors showed by sequence alignment that amino acids 542 to 548 of SEQ ID NO : 1 are relevant for the ability to increase the plasma levels of anti-α-MSH-reactive antibodies.

Accordingly, in one embodiment, derivatives, fragments or variants of comprise the amino acids 542 to 548 from SEQ ID NO : 1 with the amino acid sequence 'RWTGIPV' (referenced under SEQ ID NO: 2), preferably 80%, 81%, 82%, 83%, 84%, 85%, 86%, 87%, 88%, 89%, 90%, 91%, 92%, 93%, 94%, 95%, 96%, 97%, 98%, 99% amino acid sequence identity to that sequences.

An "anti-ClpB antibody", in the context of the invention refers to an antibody that binds to the ClpB protein as defined herein above.

Preferably, the anti-ClpB antibody cross-reacts with α-MSH as defined above.

Thus, in one embodiment, the anti-ClpB antibody is also an anti-α-MSH antibody.

An "antibody" or "immunoglobulin" (Ig) may be a natural or conventional antibody in which two heavy chains are linked to each other by disulfide bonds and each heavy chain is linked to a light chain by a disulfide bond. There are two types of light chain, lambda (I) and kappa (k). There are five main heavy chain classes (or isotopes) which determine the functional activity of an antibody molecule: IgM, IgD, IgG, IgA and IgE. Each chain contains distinct sequence domains. The light chain includes two domains or regions, a variable domain (VL) and a constant domain (CL). The heavy chain includes four domains, a variable domain (VH) and three constant domains (CH1, CH2 and CH3, collectively referred to as CH). The variable regions of both light (VL) and heavy (VH) chains determine binding recognition and specificity to the antigen. The constant region domains of the light (CL) and heavy (CH) chains confer important biological properties such as antibody chain association, secretion, trans-placental mobility, complement binding, and binding to Fc receptors (FcR). The Fv fragment is the N-terminal part of the Fab fragment of an immunoglobulin and consists of the variable portions of one light chain and one heavy chain. The specificity of the antibody resides in the structural complementarity between the antibody combining site and the antigenic determinant. Antibody combining sites are made up of residues that are primarily from the hypervariable or complementarity determining regions (CDRs). Occasionally, residues from nonhypervariable or framework regions (FR) influence the overall domain structure and hence the combining site. Complementarity Determining Regions or CDRs refer to amino acid sequences which together define the binding affinity and specificity of the natural Fv region of a native immunoglobulin binding site. The light and heavy chains of an immunoglobulin each have three CDRs, designated CDR1-L, CDR2-L, CDR3-L and CDR1-H, CDR2-H, CDR3-H, respectively. A conventional antibody antigen-binding site, therefore, includes six CDRs, comprising the CDR set from each of a heavy and a light chain V region.

"Framework Regions" (FRs) refer to amino acid sequences interposed between CDRs, *i.e.* to those portions of immunoglobulin light and heavy chain variable regions that are relatively conserved among different immunoglobulins in a single species. The light and heavy chains of an immunoglobulin each have four FRs, designated FR1-L, FR2-L, FR3-L, FR4-L, and FR1-H, FR2-H, FR3-H, FR4-H, respectively.

As used herein, a "human framework region" is a framework region that is substantially identical (about 85%, or more, in particular 90%, 95%, 97%, 99% or 100%) to the framework region of a naturally occurring human antibody.

As used herein, the term "antibody" denotes conventional antibodies and fragments thereof, as well as single domain antibodies and fragments thereof, in particular variable heavy chain of single domain antibodies, and chimeric, humanised, bispecific or multispecific antibodies.

As used herein, antibody or immunoglobulin also includes "single domain antibodies" which have been more recently described and which are antibodies whose complementary determining regions are part of a single domain polypeptide. Examples of single domain antibodies include heavy chain antibodies, antibodies naturally devoid of light chains, single domain antibodies derived from conventional four-chain antibodies, engineered single domain antibodies. Single domain antibodies may be derived from any species including, but not limited to mouse, human, camel, llama, goat, rabbit and bovine. Single domain antibodies may be naturally occurring single domain antibodies known as heavy chain antibody devoid of light chains. In particular, *Camelidae* species, for example camel, dromedary, llama, alpaca and guanaco, produce heavy chain antibodies naturally devoid of light chain. Camelid heavy chain antibodies also lack the CH1 domain.

The variable heavy chain of these single domain antibodies devoid of light chains are known in the art as "VHH" or "nanobody". Similar to conventional VH domains, VHHs contain four FRs and three CDRs. Nanobodies have advantages over conventional antibodies: they are about ten times smaller than IgG molecules, and as a consequence properly folded functional nanobodies can be produced by *in vitro* expression while achieving high yield. Furthermore, nanobodies are very stable, and resistant to the action of proteases. The properties and production of nanobodies have been reviewed by Harmsen and De Haard HJ (Appl. Microbiol. Biotechnol. 2007 Nov; 77(1): 13-22).

The antibody of the invention may be a polycloncal antibody, in particular a polyclonal antibody isolated from an obese individual, or a monoclonal antibody. Preferably, the antibody is a human antibody or rabbit antibody, more preferably a human antibody.

The antibody may be a IgM, IgD, IgG, IgA and IgE antibody, in particular an IgG or an IgM antibody, more particularly an IgG antibody.

The antibody may be a monoclonal antibody. Said monoclonal antibody may be humanized. In another example the antibody may be an a fragment selected from the group consisting of Fv, Fab, F(ab')2, Fab', dsFv, (dsFv)2, scFv, sc(Fv)2, diabodies and VHH.

The term "monoclonal antibody" or "mAb" as used herein refers to an antibody molecule of a single amino acid composition that is directed against a specific antigen, and is not to be construed as requiring production of the antibody by any particular method. A monoclonal antibody may be produced by a single clone of B cells or hybridoma, but may also be recombinant, i.e. produced by protein engineering.

The term "chimeric antibody" refers to an engineered antibody which in its broadest sense contains one or more regions from one antibody and one or more regions from one or more other antibody(ies). In particular, a chimeric antibody comprises a VH domain and a VL domain of an antibody derived from a non-human animal, in association with a CH domain and a CL domain of another antibody, in particular a human antibody. As the non-human animal, any animal such as mouse, rat, hamster, rabbit or the like can be used. A chimeric antibody may also denote a multispecific antibody having specificity for at least two different antigens. In an embodiment, a chimeric antibody has variable domains of mouse origin and constant domains of human origin

The term "humanised antibody" refers to an antibody which is initially wholly or partially of non-human origin and which has been modified to replace certain amino acids, in particular in the framework regions of the heavy and light chains, in order to avoid or minimize an immune response in humans. The constant domains of a humanized antibody are most of the time human CH and CL domains. In an embodiment, a humanized antibody has constant domains of human origin.

"Fragments" of (conventional) antibodies comprise a portion of an intact antibody, in particular the antigen binding region or variable region of the intact antibody. Examples of antibody fragments include Fv, Fab, F(ab')2, Fab', dsFv, (dsFv)2, scFv, sc(Fv)2, diabodies, bispecific and multispecific antibodies formed from antibody fragments. A fragment of a conventional antibody may also be a single domain antibody, such as a heavy chain antibody or VHH.

The term "Fab" denotes an antibody fragment having a molecular weight of about 50,000 and antigen binding activity, in which about a half of the N-terminal side of H chain and the entire L chain, among fragments obtained by treating IgG with a protease, papaine, are bound together through a disulfide bond.

The term "F(ab')2" refers to an antibody fragment having a molecular weight of about 100,000 and antigen binding activity, which is slightly larger than the Fab bound via a disulfide bond of the hinge region, among fragments obtained by treating IgG with a protease, pepsin.

The term "Fab'" refers to an antibody fragment having a molecular weight of about 50,000 and antigen binding activity, which is obtained by cutting a disulfide bond of the hinge region of the F(ab')2.

A single chain Fv ("scFv") polypeptide is a covalently linked VH::VL heterodimer which is usually expressed from a gene fusion including VH and VL encoding genes linked by a peptide-encoding linker. The human scFv fragment of the invention includes CDRs that are held in appropriate conformation, in particular by using gene recombination techniques. Divalent and multivalent antibody fragments can form either spontaneously by association of monovalent scFvs, or can be generated by coupling monovalent scFvs by a peptide linker, such as divalent sc(Fv)₂. "dsFv" is a VH::VL heterodimer stabilised by a disulphide bond. "(dsFv)2" denotes two dsFv coupled by a peptide linker.

The term "bispecific antibody" or "BsAb" denotes an antibody which combines the antigen-binding sites of two antibodies within a single molecule. Thus, BsAbs are able to bind two different antigens simultaneously. Genetic engineering has been used with increasing frequency to design, modify, and produce antibodies or antibody derivatives with a desired set of binding properties and effector functions as described for instance in EP 2 050 764 A1.

The term "multispecific antibody" denotes an antibody which combines the antigen-binding sites of two or more antibodies within a single molecule.

The term "diabodies" refers to small antibody fragments with two antigen-binding sites, which fragments comprise a heavy-chain variable domain (VH) connected to a light-chain variable domain (VL) in the same polypeptide chain (VH-VL). By using a linker that is too short to allow pairing between the two domains on the same chain, the domains are forced to pair with the complementary domains of another chain and create two antigen-binding sites.

In one embodiment the vaccine or immunogenic composition is for increasing weight gain in the subject.

Weight gain refers in the context of the invention to the weight gained after at least 16 days, for example 16, 17, 18, 19 or 20 days, preferably 20 days after the first administration of the composition comprising a bacterial ClpB protein.

Weight gain further refers to at least 2% increase of weight compared to the weight before the first administration of the vaccine or immunogenic composition comprising a bacterial ClpB protein, preferably 3% weight gain, more preferably 5% weight gain.

The term "treating", "treatment" and "preventing" is defined above.

A disease or disorder related to reduced appetite and/or anxiety is selected from the group consisting of anorexia nervosa (AN), bulimia nervosa (BN), cachexia and wasting diseases, preferably from the group consisting of anorexia nervosa (AN) and bulimia nervosa (BN).

"Anxiety" and/or "anxiety disorders" have been defined in the section *"Subject"* above.

Also provided is a method of immunization or vaccination of a subject, said method comprising administering to a subject in need thereof a vaccine or an immunogenic composition comprising a bacterial ClpB protein.

In particular, said method is for treating or reducing the chances of occurrence of a disease or disorder related to reduced appetite and/or anxiety in a subject

A subject in need thereof is a subject suffering from reduced appetite and/or anxiety and/or a disease or disorder related to reduced appetite and/or anxiety or a subject susceptible to suffer from reduced appetite and/or anxiety and/or a disease or disorder related to reduced appetite and/or anxiety.

Also provided is the use of a composition comprising a bacterial ClpB protein for the manufacture of a vaccine or immunogenic composition for the immunization against a disease or disorder related to reduced appetite and/or anxiety.

In particular, said composition is for the treatment or prevention of a disease or disorder related to reduced appetite and/or anxiety.

The vaccine or immunogenic composition may be used, for example, to stimulate appetite, wherein the reduced appetite is due to anorexia, anorexia nervosa, bulimia nervosa, cachexia or wasting diseases, in particular anorexia, anorexia nervosa or bulimia nervosa.

In one embodiment, the vaccine or immunogenic composition comprising a bacterial ClpB protein has an appetite stimulating function.

In a further embodiment the vaccine or immunogenic composition comprising a bacterial ClpB protein is for increasing appetite in said subject.

A compound has an "appetite stimulating function" if a subject to whom said compound was administered shows a stimulation of food intake and/or calorie intake resulting in an increase of food intake.

The appetite stimulating function of the vaccine or immunogenic composition in the context of the invention may be tested in administration experiments by injecting the composition intraperitoneally in free feeding rats. After administration of the composition or compound the food intake of the animals is measured. Food intake can be measured for example at 1 day to 30 days, in particular at 2, 4, 6, 8, 10, 12, 14, 16, 18, 20, 22, 24, 26, 28, 30 days.

The food intake may be measured by measuring the numbers of meals and/or meal size.

The stimulation of appetite may be measured using for instance a visual analog scale for measuring appetite, feeling of hunger or satiety level. In a preferred embodiment of the invention, the stimulation is at least 2% higher compared to prior to the treatment, such as 3% higher, more preferably 5% higher or even more preferably 6%, 7%, 8%, 9% or 10% higher compared to prior to the treatment.

The vaccine or immunogenic composition comprising a bacterial ClpB protein preferably has an appetite stimulating function and stimulates food intake. Due to this function the composition may be called in one embodiment interchangeably orexigenic or appetite stimulant because orexigenics increase the appetite of a subject.

"Food intake" has been defined above.

The vaccine or immunogenic composition comprising a bacterial ClpB protein may further reduce anxiety.

In one aspect the present invention relates to stimulating weight gain or maintaining a stable body-weight by administering the composition comprising an anti-ClpB antibody.

A low body weight might be considered in certain cultures as a physical appearance that is considered to be less attractive.

Therefore, the present invention also relates to a non-therapeutic method of increasing appetite in a subject, comprising administering to said subject an effective amount of a composition comprising an anti-ClpB antibody.

The invention further provides a non-therapeutic method of altering food intake and/or the weight of a subject, comprising administering to said subject an effective amount of a composition comprising an anti-ClpB antibody.

In one embodiment, it is envisaged that, according to the present invention, the composition can be administered to any subject suffering from weight loss, reduced appetite or even loss of appetite.

In a further embodiment, said subject may be characterized by a BMI < 17.

The immunization against a disease or disorder related to reduced appetite and/or anxiety using the vaccine or immunogenic composition comprising a bacterial ClpB protein may be achieved using any administration method known in the art, as described below in the section *"compositions".*

Preferably, for the vaccine or immunogenic composition comprising a bacterial ClpB protein, administration may be achieved using any of the administration methods described herein, more preferably using intravenous or subcutaneous administration, most preferably using subcutaneous administration methods.

In one embodiment, the invention relates to a composition comprising a bacterial ClpB protein for use in the treatment or prevention of a disease or disorder related to reduced appetite and/or anxiety, wherein said treatment or prevention of a disease or disorder relates to reduced appetite and/or anxiety is achieved by immunizing the subject using said composition.

The invention relates as well to the corresponding method of treatment or reduction of the chances of occurrence of a disease or disorder related to reduced appetite and/or anxiety in a subject, comprising immunizing the subject in need thereof with a composition comprising a bacterial ClpB protein.

A subject in need thereof is as previously defined.

Also provided is the use of a composition comprising a bacterial ClpB protein for the manufacture of a vaccine or immunogenic composition for the treatment or prevention of a disease or disorder related to reduced appetite and/or anxiety, wherein said treatment or prevention of a disease or disorder relates to reduced appetite and/or anxiety is achieved by immunizing the subject using said composition.

In one embodiment, the treatment or prevention of reduced appetite and/or anxiety may be achieved by directly administering an anti-ClpB antibody.

In another embodiment vaccine or immunogenic compositions can also comprise combinations of bacterial ClpB proteins disclosed herein.

Methods of obtaining a vaccine composition or immunogenic composition are well known in the art. Generally, such methods involve extracting proteins from bacterial preparations using techniques such as sonication, proteolytic digestion, heat treatment, freeze-thaw treatment, osmotic shock treatment etc. Examples of artificial bacterial preparations include protein preparations either in part or entirely obtained by synthetic or recombinant methods.

A typical dose for a vaccine or immunogenic composition comprising bacterial ClpB can be, for example, in the range of from about 0.01 nmol/kg to about 1000 nmol/kg; however, doses below or above this exemplary range are envisioned, especially considering the aforementioned factors. In particular, said dose is from about 0.1 nmol/kg to about 100 nmol/kg of total body weight, preferably from about 0.1 nmol/kg to about 20 nmol/kg, more particularly from 0.1 nmol/kg to about 10 nmol/kg, in particular 0.1, 0.2, 0.3, 0.4, 0.5, 0.6, 0.7, 0.8, 0.9, 1, 2, 3, 4, 5, 6, 7, 8, 9, 10 nmol/kg body weight.

The preparation of such compositions is further described below in the part "compositions".

### Compositions

Preferably, the composition used in the context of the invention for the treatment of obesity is a pharmaceutical composition.

Pharmaceutical compositions in the context of this invention preferably contain an "effective amount" or "therapeutically effective amount," or a "prophylactically effective amount," of a composition of the invention.

An "effective amount" or a "therapeutically effective amount" refers to an amount effective, at dosages and for periods of time necessary, to achieve the desired therapeutic result. An effective amount or a therapeutically effective amount of the compositions used in the context of the invention can vary according to factors such as the disease state, age, sex, and weight of the subject. An effective amount or therapeutically effective amount is also one in which any toxic or detrimental effects of the composition outweighed by the therapeutically beneficial effects.

A "prophylactically effective amount" refers to an amount effective, at dosages and for periods of time necessary, to achieve the desired prophylactic result. Typically, since a prophylactic dose is used in subjects prior to or at an earlier stage of disease, a prophylactically effective amount will be less than a therapeutically effective amount.

An effective amount or a therapeutically effective amount is at least the minimal dose, but less than a toxic dose, of an active agent which is necessary to impart therapeutic benefit to a subject. Stated another way, such an amount for treating overweight and/or obesity, for example, is an amount that induces, ameliorates, or otherwise causes an improvement in the state of the subject, for example decreasing the food intake. The pharmaceutical compositions according to the invention may be administered orally in the form of a suitable pharmaceutical unit dosage form. The pharmaceutical compositions of the invention may be prepared in many forms that include tablets, hard or soft gelatin capsules, aqueous solutions, suspensions, and liposomes and other slow-release formulations, such as shaped polymeric gels.

The mode of administration and dosage forms are closely related to the properties of the therapeutic agents or compositions which are desirable and efficacious for the given treatment application. Suitable dosage forms include, but are not limited to, oral, intravenous, rectal, sublingual, mucosal, nasal, ophthalmic, subcutaneous, intramuscular, transdermal, spinal, intrathecal, intra-articular, intra-arterial, sub-arachnoid, bronchial, and lymphatic administration, and other dosage forms for systemic delivery of active ingredients.

Pharmaceutical compositions of the invention may be administered by any method known in the art, including, without limitation, transdermal (passive via patch, gel, cream, ointment or iontophoretic); intravenous (bolus, infusion); subcutaneous (infusion, depot); transmucosal (buccal and sublingual, e.g., orodispersible tablets, wafers, film, and effervescent formulations; conjunctival (eyedrops); rectal (suppository, enema)); or intradermal (bolus, infusion, depot).

Oral liquid pharmaceutical compositions may be in the form of, for example, aqueous or oily suspensions, solutions, emulsions, syrups or elixirs, or may be presented as a dry product for constitution with water or other suitable vehicle before use. Such liquid pharmaceutical compositions may contain conventional additives such as suspending agents, emulsifying agents, non-aqueous vehicles (which may include edible oils), or preservatives.

Pharmaceutical compositions of the invention may also be formulated for parenteral administration (e.g., by injection, for example, bolus injection or continuous infusion) and may be presented in unit dosage form in ampoules, pre-filled syringes, small volume infusion containers or multi-dose containers with an added preservative. The pharmaceutical compositions may take such forms as suspensions, solutions, or emulsions in oily or aqueous vehicles, and may contain formulating agents such as suspending, stabilizing and/or dispersing agents. Alternatively, the pharmaceutical compositions of the invention may be in powder form, obtained by aseptic isolation of sterile solid or by lyophilization from solution, for constitution with a suitable vehicle, e.g. sterile, pyrogen-free water, before use.

Pharmaceutical compositions suitable for rectal administration wherein the carrier is a solid are most preferably presented as unit dose suppositories. Suitable carriers include cocoa butter and other materials commonly used in the art, and the suppositories may be conveniently formed by admixture of the pharmaceutical composition with the softened or melted carrier(s) followed by chilling and shaping in molds.

For administration by inhalation, the pharmaceutical compositions according to the invention are conveniently delivered from an insufflator, nebulizer or a pressurized pack or other convenient means of delivering an aerosol spray. Pressurized packs may comprise suitable propellant such as dichlorodifluoromethane, trichlorofluoromethane, dichlorotetrafluoroethane, carbon dioxide or other suitable gas. In the case of a pressurized aerosol, the dosage unit may be determined by providing a valve to deliver a metered amount. Alternatively, for administration by inhalation or insufflation, the pharmaceutical compositions of the invention may take the form of a dry powder composition, for example, a powder mix of the pharmaceutical composition and a suitable powder base such as lactose or starch. The powder composition may be presented in unit dosage form in, for example, capsules or cartridges or, e.g., gelatin or blister packs from which the powder may be administered with the aid of an inhalator or insufflator.

For intra-nasal administration, the pharmaceutical compositions of the invention may be administered via a liquid spray, such as via a plastic bottle atomizer. Typical of these are the Mistometerg (isoproterenol inhaler-Wintrop) and the Medihaler® (isoproterenol inhaler-Riker).

Pharmaceutical compositions of the invention may also contain excipient such as flavorings, colorings, anti-microbial agents, or preservatives.

The administration regimen may be for instance for a period of at least 2, 3, , 5, 6, 7, 8, 9, 10, 15, 20, 25, 30, 35, 40, 45, 50, 55, 60, 65, 70, 75, 80, 85, 90, 95, 100 days.

The dose range depends on the composition to be administered and is defined above.

As is well known in the medical arts, dosages for any one subject depend on many factors, including the patient's size, body surface area, age, the particular compound to be administered, sex, time and route of administration, general health, and other drugs being administered concurrently.

As regards to the vaccine and immunogenic compositions, forms suitable for injectable use include sterile aqueous solutions or dispersions and sterile powders for the extemporaneous preparation of sterile injectable solutions or dispersions In all cases the form must be sterile and must be fluid to the extent that easy syringability exists. It must be stable under the conditions of manufacture and storage and must be preserved against the contaminating action of microorganisms, such as bacteria and fungi. The carrier can be a solvent or dispersion medium containing, for example, water, ethanol, polyol (e.g., glycerol, propylene glycol, and liquid polyethylene glycol, and the like), suitable mixtures thereof, and/or vegetable oils. Proper fluidity may be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

For parenteral administration in an aqueous solution, for example, the solution should be suitably buffered if necessary and the liquid diluent first rendered isotonic with sufficient saline or glucose. These particular aqueous solutions are especially suitable for intravenous, intraarterial, intramuscular, subcutaneous, intratumoral and intraperitoneal administration. In this connection, sterile aqueous media that can be employed will be known to those of skill in the art in light of the present disclosure. For example, one dosage may be dissolved in 1 ml of isotonic NaCl solution and either added to 1000 ml of hypodermoclysis fluid or injected at the proposed site of infusion, (see for example, (see for example, Remington: The Science and Practice of Pharmacy, 21st Edition, Lippincot and Williams, 2005). Some variation in dosage will necessarily occur depending on the condition of the subject being treated. Moreover, for human administration, preparations should meet sterility, pyrogenicity, general safety and purity standards as required by FDA Office of Biologics standards.

Sterile injectable solutions are prepared by incorporating the active compounds in the required amount in the appropriate solvent by filtered sterilization.

As used herein, 'carrier' includes, without limitation, solvents, dispersion media, vehicles, coatings, diluents, antibacterial and antifungal agents, isotonic and absorption delaying agents, buffers, carrier solutions, suspensions, colloids, liposomes and virosomes such as those described in Bomsel et al (2011) Immunity 34: 269-280. The use of such media and agents for pharmaceutical active substances is well known in the art.

The phrase 'pharmaceutically-acceptable' or 'pharmacologically-acceptable' refers to molecular entities and compositions that do not produce an allergic or similar untoward reaction when administered to a human. The preparation of an aqueous composition that contains a protein as an active ingredient is well understood in the art. Typically, such compositions are prepared as injectables, either as liquid solutions or suspensions; solid forms suitable for solution in, or suspension in, liquid prior to injection can also be prepared.

In a particular embodiment of the invention, the immunogenic or vaccine composition further comprises one or several adjuvant(s).

The term "adjuvant" as used herein denotes a product which, added to the content of an immunogenic composition, in particular to a vaccine, increases the intensity of the immune reaction induced in the host to which said composition is administered. An adjuvant may in particular increase the quantity of specific antibodies which said mammal is capable of producing after administration of said composition and thus increases the efficiency of immunization.

Preferably, the adjuvant(s) is devoid of side effect in a human host.

Adjuvants include any compound or compounds that act to increase a protective immune response. Adjuvants can include for example, emulsifiers; muramyl dipeptides; avridine; aqueous adjuvants such as aluminum hydroxide; oxygen-containing metal salts; chitosan-based adjuvants, and any of the various saponins, oils, and other substances known in the art, such as Ampfaigen, LPS, bacterial cell wall extracts, bacterial DNA, CpG sequences, synthetic oligonucleotides and combinations thereof (Schijns et al (2000) Curr. Opin. Immunol, 12:456), Mycohacterialplilei (phlei) cell wall extract (CWE) (U.S. Patent No. 4,744,984), M. phlei DNA (M-D A), and M-DNA-M phlei ceil wall complex (MCC), heat-labile enterotoxin (LT), cholera toxin (CT), cholera toxin B subunit (CTB), polymerised liposomes, mutant toxins, e.g. LTK63 and LTR72, microcapsules, interleukins (e.g. IL-1β, IL-2, IL-7, IL-12, INFγ), GM-CSF, MDF derivatives, CpG oligonucleotides, LPS, MPL, phosphophazenes, Adju-Phos®, glucan, antigen formulation, liposomes, DDE, DHEA, DMPC, DMPG, DOC/Alum Complex, Freund's incomplete adjuvant, ISCOMs®, LT Oral Adjuvant, muramyl dipeptide, monophosphoryl lipid A, muramyl tripeptide, and phospatidylethanolamine. Compounds which can serve as emulsifiers include natural and synthetic emulsifying agents, as well as anionic, cationic and nonionic compounds. Oxygen-containing metal salts include salts of Al, K, Ca, Mg, Zn, Ba, Na, Li, B, Be, Fe, Si, Co, Cu, Ni, Ag, Au, and Cr which are sulphates, hydroxides, phosphates, nitrates, iodates, bromates, carbonates, hydrates, acetates, citrates, oxalates, and tartrates, and mixed forms thereof, including aluminium hydroxide, aluminium phosphate, aluminium sulphate, potassium aluminium sulphate, calcium phosphate, Maalox (mixture of aluminium hydroxide and magnesium hydroxide), beryllium hydroxide, zinc hydroxide, zinc carbonate, zinc chloride, and barium sulphateAmong the synthetic compounds, anionic emulsifying agents include, for example, the potassium, sodium and ammonium sails of lauric and oleic acid, the calcium., magnesium and aluminum salts of fatty acids, and organic sulfonates such as sodium lauryl sulfate. Synthetic cationic agents include, for example, cetyltrhethylarnmonlum bromide, while synthetic nonionic agents are exemplified by glycerylesters (e.g., glyceryl monostearate), polyoxyethylene glycol esters and ethers, and the sorbitan fatty acid esters (e.g., sorbitan monopalmitate) and their polyoxyethylene derivatives (e.g., polyoxyethylene sorbitan. monopalmitate). Natural emulsifying agents include acacia, gelatin, lecithin and cholesterol.

Other suitable adjuvants can be formed with an oil component, such as a single oil, a mixture of oils, a water-in-oil emulsion, or an oil-in- water emulsion. The oil can be a mineral oil, a vegetable oil, or an animal oil. Mineral oils are liquid hydrocarbons obtained from petrolatum via a distillation technique, and are also referred to in the art as liquid paraffin, liquid petrolatum, or white mineral oil. Suitable animal oils include, for example, cod liver oil, halibut oil, menhaden oil, orange roughy oil and shark liver oil, all of which are available commercially. Suitable vegetable oils, include, for example, canola oil, almond oil, cottonseed oil, com oil, olive oil, peanut oil, safflower oil, sesame oil, soybean oil, and the like. Freund's Complete Adjuvant (FCA) and Freund's incomplete Adjuvant (FIA) are two common adjuvants that are commonly used in vaccine preparations, and are also suitable for use in the present invention. Both FCA and FIA are water-in-mineral oil emulsions; however, FCA also contains a killed Mycobacterium sp. Particularly preferred adjuvants for mucosal vaccines include galactosyl ceramide (GalCer), as described in Lee et al (2011) Vaccine 29: 417-425.

Immunomodulatory cytokines can also be used in the vaccine compositions to enhance vaccine efficacy, for example, as an adjuvant, Non-limiting examples of such cytokines include interferon alpha (IFN-a), interleukin-2 (IL-2), and granulocyte rnacrophage-colony stimulating factor (GM--CSF), or combinations thereof.

The vaccine and immunogenic compositions, may be administered by intravenous administration, intrarterially, endoscopically, intralesionally, percutaneously, subcutaneously, intramuscular, intrathecally, intraorbitally, intradermally, intraperitoneally, transtracheally, subcuticularly, by intrasternal injection, intravenous, intrarterial, intradermal, transdermal, intramuscular, intranasal, subcutaneous, percutaneous, intratracheal, intraperitoneal, by inhalation or intranasal spraying, by endotracheal route and the like. Administration of the compositions can be by infusion or injection (e.g., intravenously, intramuscularly, intracutaneousiy, subcutaneously, intrathecal, intraduodenally, intraperitoneally, and the like). Additionally, said compositions can be administered by "needle-free" delivery systems.

The injections can be split into multiple injections, with such split inoculations administered preferably substantially concurrently. When administered as a split inoculation, the dose of the immunogen is preferably, but not necessarily, proportioned equally in each separate injection. If an adjuvant is present in the vaccine composition, the dose of the adjuvant is preferably, but not necessarily, proportioned equally in each separate injection. The separate injections for the split inoculation are preferably administered substantially proximal to each other on the patient's body. In some preferred aspects, the injections are administered at least about 1 cm apart from each other on the body. In some preferred aspects, the injections are administered at least about 2.5 cm apart from each other on the body. In highly preferred aspects, the injections are administered at least about 5 cm apart from each other on the body, in some aspects, the injections are administered at least about 10 cm apart from each other on the body, in some aspects, the injections are administered more than 10 cm apart from each other on the body, for example, at least about 12,5. 15, 17.5, 20, or more cm apart from each other on the body.

Various alternative pharmaceutical delivery systems may be employed. Non-limiting examples of such systems include liposomes and emulsions. Certain organic solvents such as dimethylsulfoxide also may he employed. Additionally, the vaccine compositions may be delivered using a sustained-release system, such as semipermeable matrices of solid polymers containing the protein. The various sustained-release materials available are well known by those skilled in the art. Sustained -release capsules may, depending on their chemical nature, release the vaccine compositions over a range of several days to several weeks to several months.

When provided prophylactically, the compositions of the invention are ideally administered to a subject in advance of evidence of a disease or disorder related to reduced appetite and/or anxiety, or in advance of any symptom of a disease or disorder related to reduced appetite and/or anxiety. The prophylactic administration of the immunogenic compositions can serve to provide protective immunity of a subject against a disease or disorder related to reduced appetite and/or anxiety or to prevent or attenuate the progression of a disease or disorder related to reduced appetite and/or anxiety. When provided therapeutically, the immunogenic compositions can serve to ameliorate and treat the reduced appetite and/or anxiety symptoms and are advantageously used as soon as possible.

Administration may be via a parenteral or non-parenteral route. Routes of administration include, e.g. intravenous, intrarterial, intradermal, transdermal, intramuscular, mucosal subcutaneous, percutaneous, intratracheal, intraperitoneal, perfusion and lavage. For example, administration is via a mucosal route, for example via a nasal, oral (via the mucosa of the digestive system), vaginal, buccal, rectal, sublingual, ocular, urinal, pulmonal or otolar (vie the ear) route.

The immunisation may include various 'unit doses.'

A unit dose need not be administered as a single injection but may comprise continuous infusion over a set period of time. A unit dose may contain from about 0.01 nmol/kg to about 1000 nmol/kg. In particular, said dose is about 0.1 nmol/kg to about 100 nmol/kg of total body weight, preferably from about 0.1 nmol/kg to about 20 nmol/kg, more particularly 0.1 nmol/kg to about 10 nmol/kg, in particular 0.1, 0.2, 0.3, 0.4, 0.5, 0.6, 0.7, 0.8, 0.9, 1, 2, 3, 4, 5, 6, 7, 8, 9, 10 nmol/kg body weight.

In one embodiment, the vaccine composition may be administered in a single daily dose, or the total daily dosage may be administered in divided doses, for example, two, three or four times daily. Furthermore, the vaccine composition may be administered in intranasal form via topical use of suitable intranasal vehicles, via transdermal routes, using those forms of transdermal skin patches well known to persons skilled in the art, by implantable pumps; or by any other suitable means of administration. To be administered in the form of a transdermal delivery system, for example, the dosage administration will, of course, be continuous rather than intermittent throughout the dosage regimen.

Vaccine administration may further comprise a prime-boost regimen. In these methods, one or more priming immunisations are followed by one or more boosting immunisations. The actual immunogenic composition can be the same or different for each immunisation and the type of immunogenic composition, the route, and formulation of the immunogens can also be varied. One useful prime-boost regimen provides for two priming immunisations, four weeks apart, followed by two boosting immunisations at 4 and 8 weeks after the last priming immunisation.

Immunisation schedules (or regimens) are well known for animals (including humans) and can be readily determined for the particular subject and composition. Hence, the compositions can be administered one or more times to the subject. Preferably, there is a set time interval between separate administrations of the immunogenic composition. While this interval varies for every subject, typically it ranges from 10 days to several weeks, and is often 2, 4, 6 or 8 weeks. For humans, the interval is typically from 2 to 6 weeks. In a particularly advantageous embodiment of the present invention, the interval is longer, advantageously about 10 weeks, 12 weeks, 14 weeks, 16 weeks, 18 weeks, 20 weeks, 22 weeks, 24 weeks, 26 weeks, 28 weeks, 30 weeks, 32 weeks, 34 weeks, 36 weeks, 38 weeks, 40 weeks, 42 weeks, 44 weeks, 46 weeks, 48 weeks, 50 weeks, 52 weeks, 54 weeks, 56 weeks, 58 weeks, 60 weeks, 62 weeks, 64 weeks, 66 weeks, 68 weeks or 70 weeks.

The immunisation regimes typically have from 1 to 6 administrations of the composition, but may have as few as 1, 2, 3, 4 or 5. The methods of inducing an immune response can also include administration of an adjuvant with the composition. In some instances, annual, biannual or other long interval (5-10 years) booster immunisation can supplement the initial immunisation protocol.

A specific embodiment of the invention provides methods of inducing an immune response against a disease or disorder related to reduced appetite and/or anxiety in a subject by administering a composition of the invention, one or more times to a subject wherein the bacterial ClpB protein is at a level sufficient to induce a specific immune response in the subject. Such immunisations can be repeated multiple times at time intervals of at least 2, 4 or 6 weeks (or more) in accordance with a desired immunisation regime.

Throughout the instant application, the term "comprising" is to be interpreted as encompassing all specifically mentioned features as well optional, additional, unspecified ones. As used herein, the use of the term "comprising" also discloses the embodiment wherein no features other than the specifically mentioned features are present (*i.e.* "consisting of").

The invention will now be described in more detail with reference to the following figures and examples.

### Sequences

SEQ ID NO: 1 shows the amino acid sequence of the chaperon protein ClpB from E. Coli K12 referenced under NCBI Reference Number NP_417083.1 and referenced under Uni-Prot entry P63284.
SEQ ID NO: 2 shows amino acids 542 to 548 of the amino acid sequence of the chaperon protein ClpB from E. Coli K12 of SEQ ID NO: 1.
SEQ ID NO: 3 shows the amino acid sequence of α-MSH from Homo sapiens referenced under Gen Pept Sequence ID, PRF: 223274.
SEQ ID NO: 4 shows the nucleic acid sequence of the nucleotide primers K12-R for amplifying orf264 of *E.coli K12.*
SEQ ID NO: 5 shows the nucleic acid sequence of the nucleotide primers K12-F for amplifying orf264 of *E.coli K12.*

### Figures

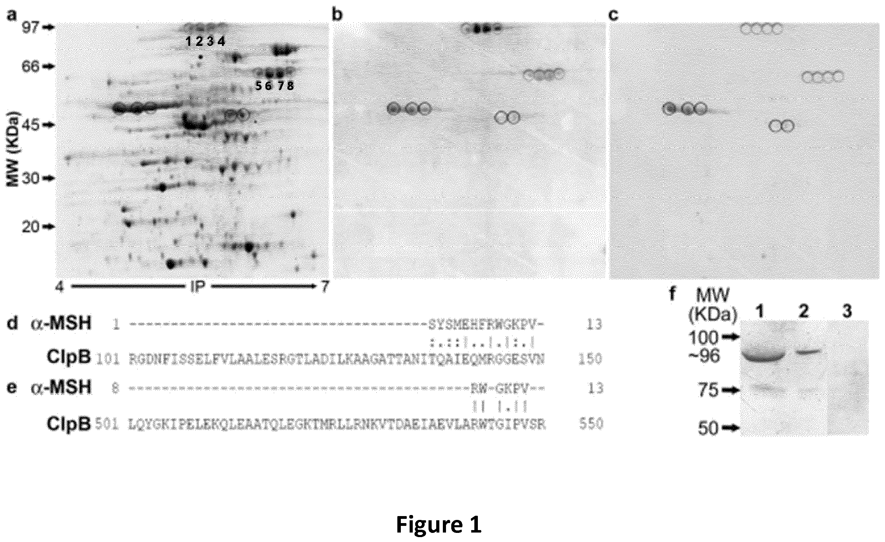

**Figure 1****: Proteomic identification of molecular mimicry between *E.coli* K12 proteins and α-MSH**
   **a.** 2 dimensional gel elecrophoresis (2DGE) of *E.coli* K12 cytoplasmic proteins. **b.** Immunoblot of *E.coli* K12 proteins detected with Rabit anti-α-MSH IgG. **c.** Immunoblot of *E.coli* K12 proteins detected with Rabit anti-α-MSH IgG preadsorbed with α-MSH. Circles 1 to 8 surround the spots specifically recognized by α-MSH IgG which were used for protein identification. Unnumbered circles indicate non specific spots. The protein identified in the spots 1-4 is ClpB. Less intensely α-MSH-like stained spots 5-8 showed highest MASCOT scores of 880, 877, 874 and 800, respectively for the 548 a. a. protein chaperonin GroEL, MW 57293 Da (YP_001732912.1). **d.** Sequences alignment between α-MSH and ClpB amino acid sequences using the Needle **e.** Stretcher programs. **f.** Western blot of recombinant ClpB revealed with Rabbit anti-α-MSH IgG. Column 1, 20 mg of ClpB, column 2, 10 mg of ClpB and column 3, 20 mg of ClpB after trypsin digestion.

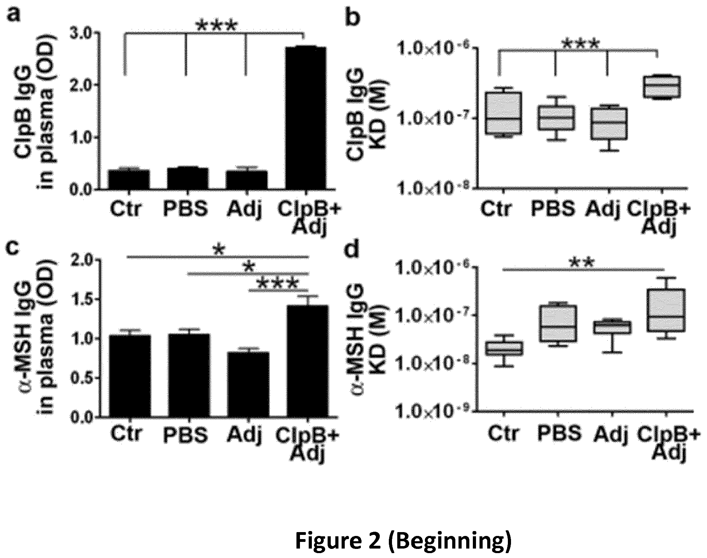

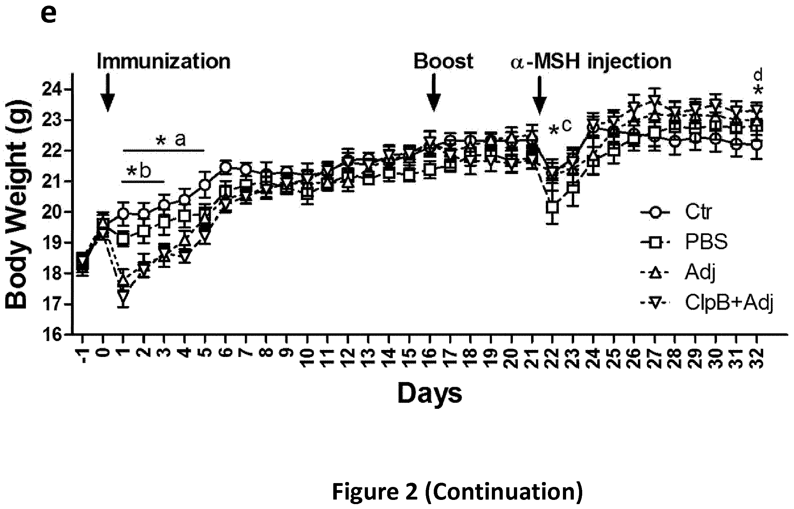

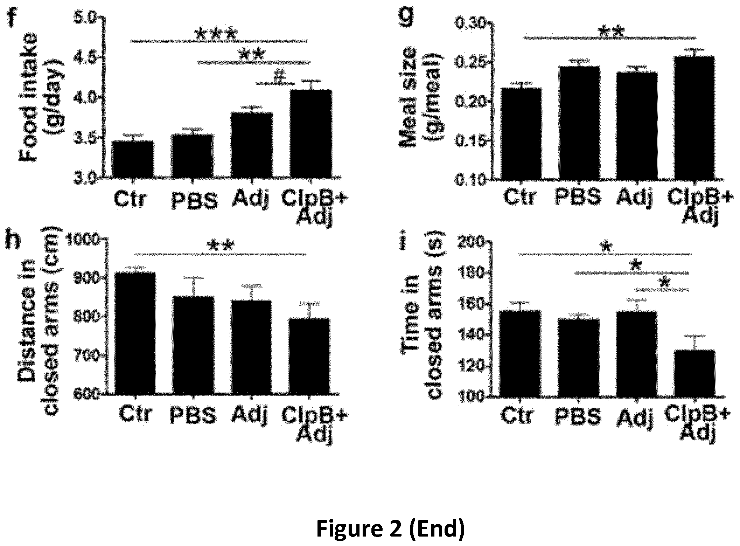

**Figure 2****: ClpB immunization in mice**
   **a.** Bar chart showing plasma levels in optical density (OD) in ELISA of ClpB-reactive total IgG. Therefore, ClpB immunized mice (ClpB+Adj) were compared with mice receiving adjuvant (Adj), PBS and controls (Ctr). **b.** Bar chart showing affinity of anti-ClpB IgG shown as the dissociation equilibrium constants (KD). **c.** Bar chart showing plasma levels of α-MSH-reactive total IgG. **d.** A Bar chart showing affinity (KD) of anti-α-MSH IgG. **e.** Graph representing body weight changes during 32 days of the study. Food intake and feeding pattern were studied during the last two weeks in the BioDAQ cages. **f.** Bar chart showing mean daily food intake and **(g)** meal size during last 10 days of the study. **h.** Bar chart representing anxiety-related behavior as expressed by distance and **(i)** time in closed arms of the O-maze. **a,b:** ANOVA *p*<0.0001, Tukey's post-tests ****p*<0.001 ClpB +Adj vs. other groups. **c.** ANOVA *p*=0.0002, Tukey's post-tests ****p*<0.001, **p*<0.05; **d:** K-W test *p*=0.003, Dunn's post-test ***p*<0.01; **e:** Two-way RM ANOVA before α-MSH injection (100 g/kg body weight, I.P.), *p*<0.0001, Bonferroni post-tests ^{*a} at least, *p*<0.05 ClpBgroup vs. Ctr, and ^{*b} at least, *p*<0.05 Adj group vs. Ctr., ^{*c}, *p*<0.05, Student's *t*-test ClpBgroup vs. PBS and ^{*d}, *p*<0.05, Student's t-test ClpB group vs. Ctr; **f:** ANOVA *p*=0.0002, Tukey's post-tests ****p*<0.001, ***p*<0.01, *^{#}p*<0.05, Student's *t*-test; **g:** ANOVA *p*=0.007, Tukey's post-tests ***p*<0.01; **h:** ***p*<0.01, Student's *t*-test; **i:** **p*<0.05, Student's *t-*test, mean±s.e.m., (n=8).

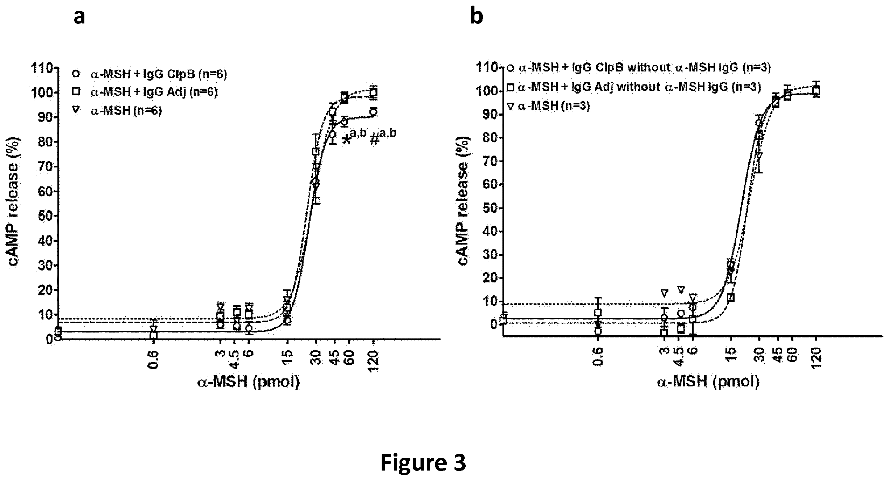

**Figure 3****: α-MSH induced cAMP release by MC4R expressing cells**
   **a.** Graph representing cAMP release in % measured with a cAMP assay in HEK 293 cells overexpressing MC4R after stimulation by α-MSH peptide alone or together with IgG (0,5 mg/ml) pooled from ClpB immunized or adjuvant control (Adj) mice. **b.** The same cAMP assay was performed with IgG from both pools which were depleted from anti-α-MSH IgG. Dose-response cAMP curves were normalized from the maximal control response. ANOVA *p*=0.005, Tukey's post-test **p*<0.05; ANOVA *p*=0.04, Student's *t*-test ^{#}*p*<0.05, ^{a}ClpB vs. α-MSH, ^{b}ClpB vs. Adj. (mean±s.e.m., n, see legends).

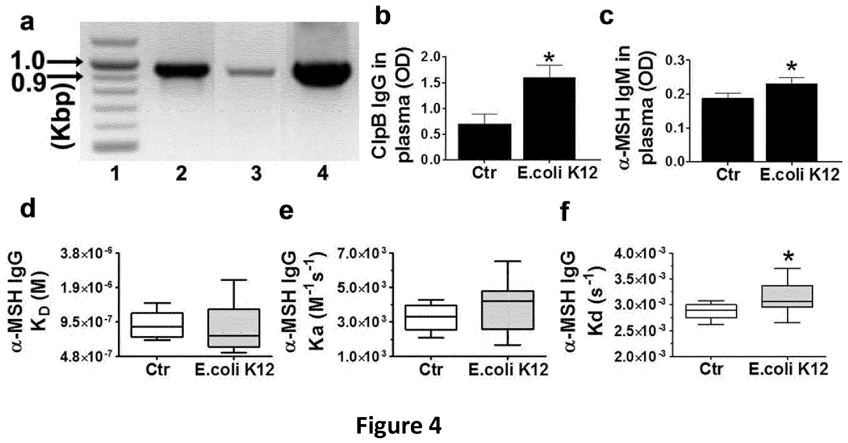

**Figure 4****: *E.coli* K12 gavage in rats**
   **a.** Electrophoresis gel showing the detection of a 0.97 kbp fragment from *E.coli K12* DNA. Columns: (1) Ready-load ™ 100 bp DNA ladder; (2) DNA extracted from cultured *E.coli K12* bacteria; DNA from rat faeces before (3) and after (4) gavage of male Wistar rats with 10⁸ *E.coli K12* in LB medium. Bar chart representing plasma levels in optical density (OD) in ELISA of anti-ClpB IgG **(b)** and anti-α-MSH IgM **(c).** Bar charts showing affinity kinetics of α-MSH IgG in *E.coli* K12 and control (Ctr) rats showing the dissociation equilibrium constants **(d),** association **(e)** and dissociation **(f)** rates. **b,c**: Student's *t*-test **p*<0.05; **f:** M.W.-test **p*<0.05, mean±s.e.m., (n=12).

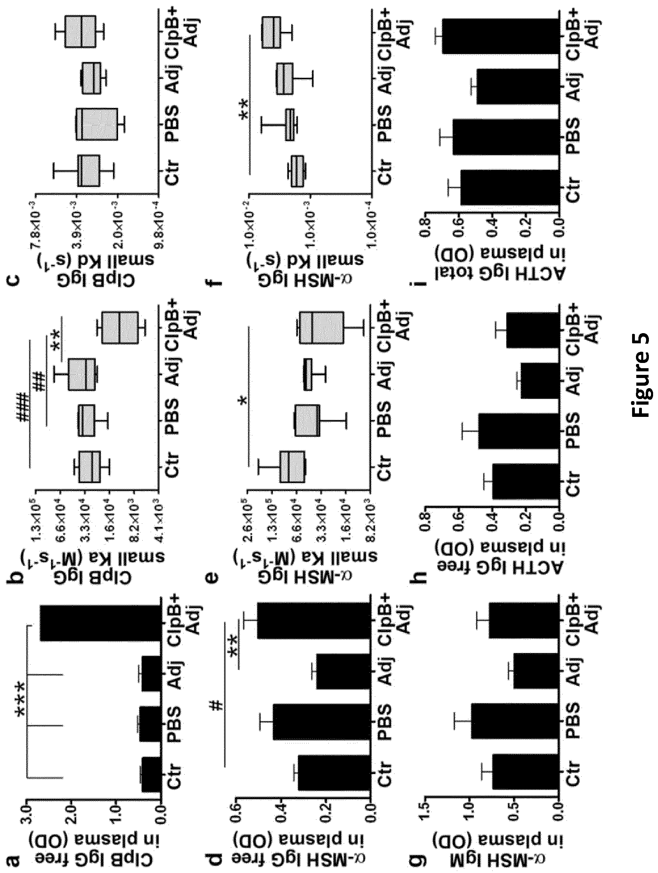

**Figure 5****: ClpB immunization in mice**
   **a.** Bar chart showing plasma levels in optical density (OD) in ELISA of free ClpB-IgG in plasma. Therefore, ClpB immunized mice (ClpB+Adj) were compared with mice receiving adjuvant (Adj), PBS and controls (Ctr). **b.** Bar chart showing the differences in the association rate constant (Ka) of anti-ClpB IgG. **c.** Bar chart showing the differences in the dissociation rate constant (Kd) of anti-ClpB IgG. **d.** Bar chart showing plasma levels in optical density (OD) in ELISA of free α-MDH-IgG in plasma. Therefore, ClpB immunized mice (ClpB+Adj) were compared with mice receiving adjuvant (Adj), PBS and controls (Ctr). **e.** Bar chart showing the differences in the association rate constant (Ka) of anti-ClpB IgG. **f.** Bar chart showing the differences in the dissociation rate constant (Kd) of anti-ClpB IgG. **g.** Bar chart showing plasma levels in optical density (OD) in ELISA of free α-MDH-IgM in plasma. Therefore, ClpB immunized mice (ClpB+Adj) were compared with mice receiving adjuvant (Adj), PBS and controls (Ctr). **h.** Bar chart showing plasma levels in optical density (OD) in ELISA of free ACTH-IgG in plasma and **i.** total ACTH-IgG in plasma. Therefore, ClpB immunized mice (ClpB+Adj) were compared with mice receiving adjuvant (Adj), PBS and controls (Ctr).

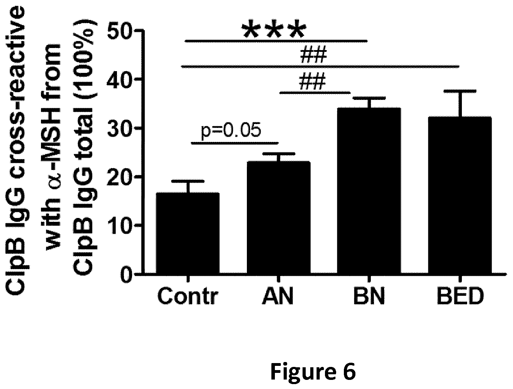

**Figure 6****: Cross reactivity of anti-ClpB IgG with α-MSH**
   Bar chart showing plasma levels (%) of anti-ClpB IgG cross-reactive with α-MSH from total anti-ClpB IgG (100%) revealed by comparison of plasma absorbed with 10⁻⁶M α-MSH with native plasma in patients with eating disorders and controls. AN-anorexia nervosa, BN-bulimia nervosa, BED-binge-eating disorder. Kruskal-Wallis test *p*=0.0006, Dunn's post-test ***p<0.001, Student's *t*-test ## p<0.01.

### Examples

The following examples demonstrate that the ClpB chaperon protein of commensal gut bacteria *E. Coli* K12 is a conformational mimetic of α-melanocyte-stimulating hormone (α-MSH), a neuropeptide involved in the regulation of energy metabolism and emotion.

The example further reveals that ClpB immunized mice show an increased production of both anti-ClpB IgG and anti-α-MSH IgG accompanied by increased food intake and body weight and lower anxiety. Furthermore, the examples show that *E.coli* K12 by gavage in rats also increased plasma levels of ClpB- and α-MSH-reactive immunoglobulins.

### Example 1

### Materials and Methods

### E.coli K12 culture and protein extraction

The bacterial strain used in this study was *E.coli* K12. This strain was available from the in house collection of the UMR 6270 CNRS Laboratory in Rouen University, France. From the frozen stocks (-80 °C), *E.coli* K12 were suspended in 250 ml of Luria broth (LB) liquid medium (MP, IIIkrich, France) and plated in LB agar to detect the contaminations. The medium containing bacteria were cultured anaerobically at 37 °C for 24 h in 50-mL tubes. To estimate the number of *E.coli* bacteria, optical density (OD) of bacterial culture was measured by a spectrophotometer at λ = 600 nm with OD 0.1 corresponding to about 10⁸ cells per ml. For protein extraction, bacteria were centrifuged at 4°C for 30 min at 4,000 g and residues were dissolved in 2 ml of trishydroxymethylaminomethane (TRIS) buffer (pH 7.4) and homogenized by sonication for 3 min at room temperature. To separate proteins from the undissolved cell fragments, the bacterial homogenate was centrifuged at 4°C for 30 min at 10,000 g. The supernatant was recovered and then ultracentrifuged at 4°C for 45 min at 60,000 g to further separate proteins into cytoplasmic (supernatant) and membrane (residues) fractions. Protein concentrations were measured using 2-D Quant Kit (GE Healthcare, Piscataway, NJ).

### Two-dimensional PAGE

For two-dimensional (2-D) polyacrylamide gel electrophoresis (PAGE), 400 µg of *E.coli K12* protein extract were used to rehydrate immobilized pH gradient (IPG) strips (pH 4-7; 18 cm; BIO-RAD, Hercules, CA). Proteins were then resolved in the first dimension by isoelectric focusing for a total of 85.000 V-h by using the IPGphor isoelectric focusing system (GE Healthcare, Piscataway, NJ). After focusing, IPG strips were incubated for 20 min in the equilibration buffer [6 mol urea/L, 30% (vol:vol) glycerol, 2% (wt:vol) sodium dodecyl sulfate (SDS), 50 mmol Tris-HCI/L pH 8.8, and 0.25% (wt:vol) bromophenol blue containing 2% (wt:vol) dithiothreitol] and then alkylated for 20 min in the equilibration buffer containing 4% (wt:vol) iodoacetamide. IPG strips were then affixed onto 10% polyacrylamide gradient gels (20 cm · 18 cm · 1 mm) for SDS-PAGE. The second dimension was performed overnight in the Ettan Daltsix vertical electrophoresis system (GE Healthcare) with 12 mA/gel at 25 C. After SDS-PAGE, the 2D gels were fixed for 2 h in 2% (vol:vol) orthophosphoric acid and in 50% (vol:vol) methanol at room temperature. Gels were then rinsed with water, and the protein spots were visualized by CBB G-250 (BIO-RAD) staining [34% (vol:vol) methanol, 17% (wt:vol) ammonium sulfate, 2% (vol:vol) orthophosphoric acid, and 0.66 g CBB G-250/L].

### Immunoblotting

Following 2D-PAGE, *E.coli* cytoplasmic proteins were transferred onto Hybond-ECL PVDF membrane (GE Healthcare) by using a dry transfer method (Trans Blot Cell, Biorad, USA) and a constant current of 0.8 mA/cm² of the membrane size for 2 h. After transfer, membranes were blocked with 5% (wt:v) milk (Regilait, France) in phosphate buffered saline (PBS; 10 mmol/L Tris, pH 8; 150 mmol/L NaCl) plus 0.05% (v:v) Tween 20. After washes, membranes were incubated overnight at 4 °C with polyclonal rabbit anti-α-MSH antisera (1:1000, Peninsula laboratories, (San Carlos, CA), following by washes and incubation with polyclonal swine anti-rabbit HRP conjugated immunoglobulins (1:3000, Dako, Trappes, France). Immunoblots were revealed by the ECL detection system (GE Healthcare) and were scanned with ImageScanner II (GE Healthcare) previously calibrated by using a greyscale marker (Kodak) and digitalized with Labscan 6.00 software (GE Healthcare). The same procedure was performed after adsorption of rabbit anti-α-MSH antisera with 10⁻⁶M of α-MSH peptide overnight at 4 °C.

### Protein identification

The protein spots of interest were excised from CBB G-250 stained 2D gels using the Ettan Spot Picker (GE Healthcare) and automated in-gel digestion of proteins was performed on the Ettan Digester (GE Healthcare). Protein extracts were then resuspended in 10 µL of 5% (vol:vol) acetonitrile/0.1% (vol:vol) formic acid and then analyzed with a nano-LC1200 system coupled to a 6340 Ion Trap mass spectrometer equipped with a nanospray source and an HPLC-chip cube interface (Agilent Technologies, Courtaboeuf, France). Briefly, peptides were enriched and desalted on a 40 nL RP-C18 trap column and separated on a Zorbax (30-nm pore size, 5-µm particle size) C18 column (43 mm long x 75 µm inner diameter; Agilent Technologies). A 9-min linear gradient (3%-80% acetonitrile in 0.1% formic acid) at a flow rate of 400 nl/min was used, and the eluent was analyzed with an Ion Trap mass spectrometer. For protein identification, MS/MS peak lists were extracted and compared with the protein databases by using the MASCOT Daemon version2.2.2 (Matrix Science) search engine. The searches were performed with the following specific parameters: enzyme specificity, trypsin; one missed cleavage permitted; no fixed modifications; variable modifications, methionine oxidation, cysteine carbamidomethylation, serine, tyrosine and threonine phosphorylation; monoisotopic; peptide charge, 2+ and 3+; mass tolerance for precursor ions, 0.5 Da; mass tolerance for fragmentations, 0.6 Da; ESI-TRAP as instrument; taxonomy, *E. coli*; National Center for Biotechnology Information (NCBI) database [NCBlnr 20120531 (18280215 sequences, 6265275233 residues)](Bethesda, MD). Protein hits were automatically validated if they satisfied one of the following criteria: identification with at least two top ranking peptides (bold and red) each with a MASCOT score of more than 54 (P< 0.01), or at least two top ranking peptides each with a MASCOT score of more than 47 (P< 0.05). To evaluate false-positive rates, all the initial database searches were performed using the "decoy" option of MASCOT. Results were considered relevant if the false-positive rate never exceeded 1%.

### Protein identification from OFFGEL

High resolution *E.coli* K12 protein separation into 24 fractions was done onto the 3100 OFFGEL fractionator using the OFFGEL pH3-10 kit (Agilent Technologies). Protein samples (400 µg) preparation and assembly of all parts of the OFFGEL systems were done according to the procedures described in the Agilent Quick start Guide. OFFGEL fractionation was performed using the standard program OG24PRO with maximum limited current parameters (8000 V, 50 µA and 200 mW) until 64 KVh was reached after 30 h. At the end of the experiment, all fractions were transferred into a 0.8 mL deep well (Themofisher, IIIkirch, France) and stored at -20 °C. Nine protein-containing fractions recovered from the central part of the OFFGEL were studied by Western blot using rabbit anti-α-MSH polyclonal antisera (Peninsula laboratories) followed by protein identification as described above.

### Immunization and behavior in mice

Animal care and experimentation were in accordance with guidelines established by the National Institutes of Health, USA and complied with both French and European Community regulations (Official Journal of the European Community L 358, 18/12/1986). Two month-old male C57BI6 mice (Janvier Labs L'Arbresle, France) were acclimated to the animal facility for with 12 h light-dark cycle, lights on at 7:00 a.m. and were kept in standard mouse holding cages (n=8) in each. Mice were fed *ad libitum* with standard pelleted rodent chow (RM1 diet, SDS, UK) with drinking water always available and were manipulated daily by gentle holding and measuring body weight. During acclimatization mice were distributed between four cages to obtain the similar mean body weight per mouse per cage. After one week of acclimatization, mice from each cage were assigned to one of four study group and received following treatments: i) Group 1, ClpB immunization (n=8): ClpB protein (Delphi Genetics) 50 µg/mouse in 200 µl of 1:1 vol. of PBS with Complete Freund's Adjuvant (CFA, Sigma), intraperitoneally (I.P.); ii) Group 2, adjuvant injection controls (n=8): 200 µl of CFA in PBS (1:1 vol., I.P.); iii) Group 3, PBS injection controls (n=8): 200 µl of PBS (I.P.) and iiii) Group 4, Intact controls (n=8): received no injections, and then all mice were returned to their holding cages. Fifteen days later, mice were given a boost immunization and the following treatments: i) Group 1 (n=8), ClpB protein (Delphi Genetics) 50 µg/mouse in 200 µl of 1:1 vol. of PBS with Incomplete Freund's Adjuvant (IFA, Sigma) I.P.; ii) Group 2 (n=8): 200 I of IFA in PBS (1:1 vol., I.P.); iii) Group 3, (n=8): 200 I of PBS (I.P.) and iiii) Group 4, (n=8): received no injections.

Next day after the boost, mice were placed individually in the BioDAQ mouse cages (Research Diets, Inc., New Brunswick, NJ) each equipped with an automatic feeding monitor. Food (SERLAB, Montataire France) and drinking water were available *ad libitum and* body weight was measured daily. After three days of acclimation to the BioDAQ cages, mice received the following treatments: Groups 1, 2 and 3 (each n=8) i.e. mice which have been immunized with ClpB; injected with adjuvants and with PBS, respectively, all received an acute injection of α-MSH peptide (Bachem), 100 mg/kg body weight in approximately 100 µl of PBS (I.P.) at 10 a.m. The control mice (n=8) received PBS only (I.P.). Feeding data was continuously monitored and analysed using the BioDAQ data viewer 2.3.07 (Research Diets). For the meal pattern analysis, the inter-meal interval was set at 300 sec.

After the feeding study, mice were placed in individual mouse holding cages with food and water available *ad libitum* and were analyzed for locomotor activity and anxiety in O-maze tests performed during two consecutive days. Two hours after the O-maze test, mice were killed by decapitation in a guillotine and trunk blood was collected into EDTA-containing tubes. Plasma was separated by centrifugation at 3,500 r.p.m. (1.4 g) for 10 min at 4 °C and stored at -80 °C before assay.

### Locomotor activity and anxiety tests

After feeding study in the BioDAQ cages, mice were analyzed for locomotor activity using a Versamax Animal Activity Monitor (AccuScan Instruments, Inc., Columbus, OH). Next day after the locomotor activity test all mice were tested for their anxiety in an elevated-O-maze. The elevated-O-maze is a variation of more commonly used elevated-plus-maze pharmacologically validated for anxiety testing in rodents (Crawley, J. N. and Bailey, K. R., Methods of behavior analysis in neuroscience, 77-101 (CRC Press, 2009)). The advantage of the O-maze is that it lacks the ambiguous central square of the traditional plus-maze. The O-maze (Med Associate, Inc., St. Albans, VT) consisted of a circular infrared platform (outer diameter 120 cm) elevated 80 cm above the floor, featuring two open and two closed segments made of grey plastic. The closed segments were enclosed by walls extending 20 cm above the surface of the maze and covered with a black infrared Plexiglas lid. Each test started by placing the mouse into one of the two closed segments. The test lasted 5 min and was recorded using a video-camera placed above the O-maze and the EthoVision video tracking software (Noldus IT, Wageningen, The Netherlands). Measurements of distance and time spent in the open and closed segments were analyzed. Between each mouse tests, the O-maze was cleaned with a 30% alcohol.

### ClpB and α-MSH autoantibody assay

Plasma levels of autoAbs reacting with ClpB, α-MSH and ACTH were measured using enzyme-linked immunosorbent assay (ELISA). Briefly, ClpB protein (Delphi Genetics), α-MSH or ACTH peptides (Bachem AG, Bubendorf, Switzerland) were coated on Maxisorp plates (Nunc, Rochester, NY) using 100 µl and a concentration of 2 µg/ml in 100 mM NaHCO3 buffer, pH 9.6 for 72 h at 4°C. Plates were washed (5 min x 3) in phosphate-buffered saline (PBS) with 0.05% Tween 200, pH 7.4, and then incubated overnight at 4°C with 100 µl of mouse or rat plasma diluted 1:200 in PBS to determine free autoAbs levels or diluted 1:200 in dissociative 3 M NaCl, 1.5 M glycine buffer, pH 8.9 to determine total autoAbs levels. The plates were washed (3x) and incubated with 100 µl of alkaline phosphatase (AP)-conjugated goat anti-rat IgG (1:2000), anti-rat IgM (1:1000), anti-mouse IgG (1:2000), or anti-mouse IgM (1:1000) all from Jackson ImmunoResearch Laboratories, Inc. (West Grove, PA). Following washing (3x), 100 µl of p-nitrophenyl phosphate solution (Sigma) was added as alkaline phosphatase substrate. After 40 min of incubation at room temperature, the reaction was stopped by adding 3N NaOH. The OD was determined at 405 nm using a microplate reader Metertech 960 (Metertech Inc., Taipei, Taiwan). Blank OD values resulting from the reading of plates without addition of plasma samples were subtracted from the sample OD values. Each determination was done in duplicate. The variation between duplicate values was less than 5%. Similar protocol was used to measure anti-ClpB IgG and IgM and anti-α-MSH IgG autoAbs in human plasma (1:400) using corresponding anti-human IgG or IgM AP-conjugated antibodies (1:2000, Jackson ImmunoResearch Laboratories).

### Absorptions of ClpB antibodies with a-MSH

Plasma samples of humans diluted 1:400 in PBS were preincubated with 10⁻⁶ M a-MSH peptide (Bachem) overnight at 4°C before adding the samples to 96-well Maxisorp plates (Nunc) coated with ClpB protein (Delphi Genetics). IgG and IgM antibodies reactive with ClpB were detected by ELISA using corresponding anti-human AP-conjugated antibodies (Jackson) as described above. Percentage of ClpB antibodies cross-reactive with a-MSH were calculated relative to levels of anti-ClpB antibodies detected without absorption in each individual plasma sample equal 100%.

### IgG purification from plasma

Extraction of plasma globulins was done by plasma acidification and separation on C-18 SEP column (Phoenix Pharmaceuticals, Burlingame, CA) according to the manufacturer instructions. In detail, 500 µl of mouse or rat plasma was mixed with 500 µl of buffer A (1% trifluoroacetic acid TFA in water). The column was activated in 1 ml of buffer B (60% acetonitrile in 1% TFA) by 3 min centrifugation with 700 rpm and rinsed 3 times with 3 ml of buffer A. Diluted plasma (1:1 in buffer A) was added to the column and the effluent (1 ml) was saved (frozen at -20°C) for further purification of IgG. Total IgG were purified from the effluents of rat plasma samples using the Melon Gel Kit (Thermo Fisher Scientific, Rockford, IL) according to the manufacturer instructions. In brief, plasma effluents diluted 1:4 in kit's purification buffer was added on washed melon gel deposited in a column. Column was spinned 1 min at 6,000 r.p.m. and the IgG containing effluent was saved and frozen at -20°C before lyophilization. Lyophilized IgG were reconstituted in the HBS-EP buffer (GE Healthcare, Piscataway, NJ).

For the cAMP experiment, IgG purified from 8 mice of the ClpB and of the adjuvant control group were combined, respectively, into two pools which were divided in two parts. One part was used directly in cAMP assay and the other was further purified using affinity chromatography for α-MSH coated on activated UltraLink beads (Pierce, Rockford, IL). The α-MSH IgG-depleted IgG effluents were saved, lyophilized and diluted in PBS to the final concentration of 0.5 mg/ml to be used in cAMP assay.

### Affinity kinetics assay

Affinity kinetics of mouse and rat IgG autoAbs for ClpB and α-MSH was determined by a biospecific interaction analysis (BIA) based on the surface plasmon resonance (SPR) phenomenon on a BIAcore 1000 instrument (GE Healthcare). α-MSH (Bachem) or ClpB protein (Delphi Genetics) were diluted 0.5 mg/ml in 10 mM sodium acetate buffer, pH 5.0 (GE Healthcare) and were covalently coupled on the sensor chips CM5 (GE Healthcare), using the amine coupling kit (GE Healthcare). All measures were performed on the same α-MSH or ClpB coated chips. For the affinity kinetic analysis, a multi-cycle method was run with five serial dilutions of each IgG sample: 3360, 1680, 840, 420 and 210 (nmol) including a duplicate of 840 nmol and a blank sample (HBS-EP buffer only). Each cycle included 2 min of analyte injection and 5 min of dissociation with flow speed 30 µl/min at 25°C. Between injections of each sample, the binding surface was regenerated with 10 mM NaOH, resulting in the same baseline level of the sensorgram. The affinity kinetic data were analyzed using BiaEvaluation 4.1.1 program (GE Healthcare). For fitting kinetic data, the Langmuir's 1:1 model was used and the sample values were corrected by subtracting the blank values.

### E.coli gavage

Two month-old female Wistar rats (n=24), body weight 220-250 g (Janvier Labs) were maintained at 24°C with a 12h light-dark cycle in a specialized animal facility; the rats were fed with standard pelleted rodent chow (RM1 diet, SDS, UK) when kept in standard holding cages during 1 week. After acclimation, rats were kept in individual metabolism cages (Tecniplast, Lyon, France) where they were fed with the same RM1 but ground chow (SDS). Drinking water was always available *ad libitum.* Body weight, food and water intakes were measured daily. *E.coli K12* were cultured as described above in LB medium. Rats were intragastrically gavaged with 4 ml of LB medium containing 10⁸ *E.coli K12* (n=12) or with 4 ml of LB medium without bacteria (Controls, n=12). The gavage procedure was carried out daily between 09:00 and 10:00 am for 21 days. After gavage rats were killed by decapitation in a guillotine and trunk blood was collected into EDTA-containing tubes. Plasma was separated by centrifugation at 3,500 r.p.m. (1.4 g) for 10 min at 4 °C and stored at -80 °C before assay.

### E.coli K12 bacterial DNA assay

The DNA was purified from rat faeces collected before starting the gavage with the QIAamp^{R} DNA Stool Mini Kit (QIAGEN, France), DNA of *E.coli K12* was also extracted from the cultures of the strain; bacteria were dissolved in water and warmed at 100 °C during 5 min, after 1 min of centrifugation at 11,000 r.p.m., the supernatant containing the DNA was stored at -20°.
Based on the published study (Kuhnert, P. et al. Appl Environ Microbiol 61, 4135-4139 (1995)) we used the following nucleotide primers, K12-R: 5'-ATCCTGCGCACCAATCAACAA-3' (SEQ ID NO: 4) and K12-F: 5'-CGCGATGGAAGATGCTCTGTA-3' (SEQ ID NO: 5) (Invitrogen Custom Primers, Cergy Pontoise, France) to amplify a region of the orf264 as a marker of *E.coli K12.* PCR was performed in a thermocycler with MicroAmp tubes (Eppendorf, Hambourg, Germany). The reaction was carried out in a 50 µl volume containing 25µL of Go Taq ^{R} Green Master Mix 2X (Promega, Madison, Wl), 1µl (20 pmol) of each primer, 21µl of bi-distilled water and 1 µl of bacterial DNA. PCR conditions were as follows: 3 min at 94°C followed by 35 cycles at 94°C for 30 s, 60°C for 30 s, and 72°C for 1.5 min. PCR products were analyzed on a 1% agarose gel (Sigma, St Louis, MO), the size of the amplification product corresponding to 0,97-kb.

### Human subjects

Plasma samples from Estonian female patients with eating disorders were used in this study. AN, BN and BED were diagnosed by a psychiatrist and a clinical psychologist according to the Diagnostic and Statistical Manual of Mental Disorders, 4th Ed. (DSM-IV). Plasma samples from healthy female volunteers served as the control group. All study patients and controls gave their informed consent for study participation. Plasma samples from both patients and controls were taken on the day of completing the EDI-2 test, instantly frozen and kept at -70°C then shipped on dry ice and stored again at -80°C until analyzed.

### In vitro cAMP assay

Stable cell line of human embryonic kidney (HEK) 293 cells expressing human MC4R was generated using a lentiviral transduction technology and purchased from Amsbio (Oxon, UK). High expression of MC4R mRNA in transfected cells was validated by RT-PCR in Amsbio and in our laboratory. The presence of the transgene in cells before each experiment was verified by the visualization at a fluorescence microscope of the green-fluorescent protein (GFP) which gene was inserted in the same with MC4R lentivector but under a different promoter. The α-MSH peptide (Bachem) was diluted in the induction buffer: PBS, 500 µM IBMX, 100 µM RO 20-1724 (Sigma-Aldrich), 20 mM MgCl₂ to the final concentrations of 2 µM, 1 µM, 750 nM, 500 nM, 250 nM, 100 nM, 75 nM, 50 nM, 10 nM corresponding to the α-MSH doses of 0.6, 3, 4.5, 6, 15, 30, 45, 60 and 120 pmol, respectively and also included one blank sample.

After unfreezing, the cells were cultured in 250 ml tissue culture flasks (BD-Falcon, Beckton-Dickison, Bedford, MA) in Dubecco's Modified Eagle Medium 4,5 g/l glucose (Eurobio, Courtaboeuf, France) supplemented with (2 mM L-glutamine; 10% FCS; 0,1 mM non-essential amino-acids; 1% penicillin-streptavidin) in humidified cell culture incubator at 37 °C, 5% CO₂ for 8-10 days. At the day of experiment, cultured MC4R HEK293 cells were treated with 0,25% trypsin-EDTA (Sigma-Aldrich) and cell pellets were resuspended in PBS to obtain about 5,000 cells par well (10 µL) in a non-treated bioluminescence white 96-microwells plate (Nunc, Roskilde, Denmark). The cyclic adenosine monophosphate (cAMP) production by MC4R expressing HEK 293 cells was measured using the bioluminescent assay cAMP-Glo™ Max Assay kit (Promega) according to the manufactures instructions. Briefly, the cells were incubated for 15 min at room temperature with different concentrations of α-MSH peptide alone or α-MSH together with mouse IgG pools from ClpB immunized or adjuvant control groups and which were added to the cells just before α-MSH. Serial dilutions of cAMP standard (provided by the kit) were assayed on the same microplate. cAMP detection solution was added to each well, then the cells were homogenized by agitation and centrifuged 2 min at 1,000 r.p.m. and then incubated for 20 min at 23°C. Kinase-Glo reagent substrate was added in each well and after 10 min of incubation at 23°C, the luminescence was read with a bioluminescence instrument (Safas Spectrometer, Monaco). Three tests for each dilution were performed in separate wells and were repeated at two separate days resulting in n=6 for each point of the cAMP activation curve when total IgG were used. After depletion of total IgG from anti-α-MSH IgG, the same experiment was performed with each α-MSH concentration and IgG measured in three separate wells.

### Statistical analysis

Data were analyzed and the graphs were plotted using the GraphPad Prism 5.02 (GraphPad Software Inc., San Diego, CA, USA). Normality was evaluated by the Kolmogorov-Smirnov test. Group differences were analyzed by the analysis of variance (ANOVA) or the non-parametric Kruskal-Wallis (K-W) test with the Tukey's or Dunn's post-tests, according to the normality results. Body weight changes were analyzed with Two-way repeated measurements (RM) ANOVA and the Bonferroni post-tests. Individual groups were compared using the Student's *t*-test or the Mann-Whitney (M-W) test according to the normality results. Pearson's or Spearman's correlation coefficients were calculated according to the normality of the variable. The cAMP production was analyzed using a non-linear regression fit (log(α-MSH) vs. normalized cAMP response) which equation was Y=100/(1+10^((LogEC50-X)*HillSlope)). Data are shown as means ± standard error of means (s.e.m), and for all test, *p*<0.05 was considered statistically significant.

### Results

### Proteomic identification of α-MSH mimicry in E.coli K12

Total protein was extracted from cultured *E.coli* K12 and separated into the cytoplasmic and membrane fractions. Two-dimensional gel electrophoresis (2-DGE) was performed with both protein fractions and for the study of α-MSH molecular mimicry, the cytoplasmic fraction was analyzed (Fig. 1a). Following 2-DGE, *E.coli* cytoplasmic proteins were transferred to a PVDF membrane and α-MSH-like proteins were immunodetected using anti-α-MSH polyclonal rabbit IgG. Use of such polyclonal antiserum provided detection of multiple α-MSH-like epitopes eventually present in *E.coli* proteins. The inventors found that about 13 protein spots were recognized by anti-α-MSH antiserum (Fig. 2b). To verify the specificity of binding, before the immunodetection, anti-α-MSH antiserum was preincubated with 10⁻⁶M α-MSH peptide resulting in complete disappearance of the immunopositive spots 1-4 and 5-8 (Fig. 1a-c), indicating that only these spots contained proteins with α-MSH-like epitopes. Based on the α-MSH absorption experiments, the α-MSH-like specific spots 1-8 (Fig. 1a) were used for protein identification by nano-LC1200 system coupled to a 6340 Ion Trap mass spectrometer equipped with a nanospray source and an HPLC-chip cube interface (Agilent Technologies). For protein identification, MS/MS peak lists were extracted and compared with the NCBI protein databases of *E.coli* using the MASCOT Daemon version 2.2.2 (Matrix Science) search engine. Protein spots 1, 2, 3 and 4 showing strongest and specific α-MSH-like staining (Fig. 1) displayed highest MASCOT scores of 819, 721, 299 and 786, respectively, for a 857 amino acid protein disaggregation chaperone or ClpB, MW 95526 Da (SEQ ID NO: 1; NP_417083.1).

To confirm the obtained results, the inventors used off-gel, an alternative strategy of protein identification. For this aim, *E.coli* cytoplasmic proteins were separated according to their isoelectric point (IP) by an OFFGEL fractionator (Agilent Technologies) followed by one-dimensional gel electrophoresis revealed by Western blot using the same anti-α-MSH polyclonal rabbit IgG preadsorbed with α-MSH. One band was specifically recognized by α-MSH antiserum and was used for protein identification as described above. The inventors found that in this band, ClpB protein was among the proteins with the highest MASCOT score (1065) and, hence, this protein was selected for further validation of its molecular mimicry with α-MSH. To analyse the amino acid sequence homology between α-MSH and ClpB, both sequences were aligned using the Emboss Needle and Stretcher programs of the Needleman-Wunsch algorithm (http://www.ebi.ac.uk/Tools/emboss/). The alignments revealed that there are two distinct sites of the ClpB protein displaying discontinuous sequence homology with α-MSH as shown using the Needle (Fig. 1d) or Stretcher (Fig. 1e) programs.

The recombinant ClpB protein was customly produced by Delphi Genetics SA, (Gosselies, Belgium) in bacterial cultures by the ClpB cDNA synthesis and cloning into an expression vector. The ClpB expected 96 kDa molecular weight was confirmed by the SDS-PAGE. Western blot of ClpB revealed with anti-α-MSH antiserum showed a single 96 kDa band, confirming that the ClpB protein contains α-MSH-like epitopes (Fig. 1f). To test if the conformation of the ClpB protein is important for its detection by anti-α-MSH antiserum, the Western blot was also performed after ClpB digestion by trypsin. No band was detected in this condition (Fig. 1f), indicating that molecular mimicry between ClpB and α-MSH should involve the conformational structure of the ClpB protein. These results indicate that at least five consecutive amino acid sequence homology, as was proposed by the molecular mimicry concept (Oldstone, M. B. Faseb J 12, 1255-1265 (1998)) is not an obligatory condition for bacterial proteins to display molecular mimicry with neuropeptides. Furthermore, the *E.coli K12* proteins that displayed such consecutive amino acid homology with α-MSH have not been identified as α-MSH mimetics by this proteomic approach. This mismatch can be related to the conformationally hidden α-MSH-like epitopes in these proteins that escaped the detection by anti- α-MSH antiserum.

### Immunization of mice with ClpB

To validate the ability of *E.coli* ClpB to induce autoAbs cross-reactive with α-MSH, the inventors immunized two month-old C57BI6 mice with the recombinant ClpB protein. First, to confirm the efficiency of immunization, the inventors assayed plasma levels anti-ClpB IgG and measured affinity kinetics between ClpB and plasma extracted IgG using the surface plasmon resonance (SPR). In ClpB immunized mice, the inventors found higher plasma levels of ClpB IgG (Fig. 2a and Fig. 5) which had lower affinities as compared to other groups (Fig. 2b and Fig. 5), which is in agreement with a recent IgG induction. Increased plasma levels of α-MSH-reactive IgG were also found in ClpB immunized mice (Fig. 2c and Fig. 5) and these IgG were also characterised by lower affinities for α-MSH as compared to controls (Fig. 2d and Fig. 5). Plasma levels of α-MSH IgM autoAbs did not significantly differ between the groups (Fig. 5), indicating that mice had been exposed to the naturally present ClpB antigen. The inventors also analyzed if ClpB immunization may induce autoAbs against adrenocorticotropic hormone (ACTH), a 39 amino acid melanocortin peptide containing the α-MSH sequence. No significant differences in plasma ACTH-reactive IgG were found among the groups (Fig. 5), further supporting that the conformational structure of the ClpB molecule was specific to stimulate α-MSH-reactive IgG.

Mice that received ClpB and CFA or CFA had lower body weight for few days after injections due to expected inflammatory response to immunization (Fig. 2e). However, at the end of the experiment, ClpB immunized mice had higher body weight (by 5%) vs. controls (Fig. 2e). The mean daily food intake as measured in the BioDAQ during last 10 days of the experiment was also higher (by 13%) in ClpB-immunized mice as compared to other groups (Fig. 2f). Increase in food intake in the ClpB group was solely due to increased meal size (Fig. 2g), since meal number did not change, indicating that the ClpB immunization interfered with the satiation rather than with hunger controlling systems. This is in agreement with a known α-MSH role to induce satiety (Azzara, A. V. et al., Physiology & Behavior 77, 411-416, (2002)). To further validate the relevance of ClpB-induced changes of α-MSH-reactive autoAbs in food intake regulation, on the day 22, ClpB, CFA and PBS groups received a single injection of α-MSH (100 µg/kg body weight, I.P.), while the control group received PBS. The following 24h food intake and body weight were found lower in non-immunized as compared to ClpB-immunized mice in which it did not differ from controls, indicating that the ClpB group had a better protection against α-MSH anorexigenic effects.

Immediately after the feeding experiments, locomotor activity and anxiety-related behavior in mice were studied in the open field and O-maze tests. The total locomotor activity and the time spent in the open vs. border areas did not significantly differ among the groups. However, in the closed arms of the O-maze, the ClpB-immunized mice moved shorter distance as compared to controls (Fig. 2h) and spent less time as compared to all other groups (Fig. 2i). This indicates a slight decrease in anxiety-related behavior in the ClpB group, since the parameters in the O-maze open arms did not significantly change. These data are in agreement with reduction of α-MSH-mediated anxiety in mice with increased anti-α-MSH IgG levels.

### Effects of mouse IgG on α-MSH-induced activation of MC4R

To validate the relevance of ClpB immunization-induced anti-α-MSH IgG to the MC4R signaling, the inventors studied in vitro effects of mouse IgG on the activation of MC4R by α-MSH. IgG (0.5 mg/ml) pooled from ClpB-immunized and from the adjuvant control groups of mice were added to HEK 293 cells overexpressing MC4R (Amsbio, Oxon, UK) before adding ten different concentrations of α-MSH peptide in PBS (from 1 nM to 2 µM, and PBS only). After 15 min of incubation, cAMP concentrations were measured in cell supernatants using the bioluminescent assay cAMP-GloTM Max Assay kit (Promega, Madison, WI). The inventors found that cAMP concentrations were lower in cells preincubated with IgG from ClpB-immunized mice as compared to α-MSH and α-MSH with IgG from the adjuvant controls with the significant reduction between 10% and 8% at the two highest α-MSH concentrations (Fig. 3a). After depletion of the pooled IgG from α-MSH-reactive IgG using α-MSH chromatography, the remaining IgG of the ClpB group did not show any effect to reduce α-MSH-induced cAMP release (Fig. 3b), indicating that anti- α-MSH IgG were responsible for the inhibition of cAMP in ClpB immunized mice. The reduction in MC4R activation may, hence, account for the increased food intake and decreased anxiety in ClpB-immunized mice. It can not, however, be excluded that ClpB-induced anti-α-MSH IgG may also interfere with α-MSH binding to other than MC4R melanocortin receptors, e.g. the MC3R (Begriche, K. et al., Journal of Biological Chemistry 2011, 286, 40771-40781) . The direct effect of ClpB on MC4R mediated cAMP production was not possible to evaluate due to the ClpB ATPase activity (Woo, K. M. et al., Journal of Biological Chemistry 1992, 267, 20429-20434) which interferes with the ATP-dependent cAMP production.

### Gavage of rats with E.coli K12

To test if without immunization, the ClpB protein naturally expressed by *E.coli* can be sensed by the immune system resulting in stimulation of ClpB- and α-MSH-reactive Ig, *E.coli* K12 were given daily by intragastric gavage to two month-old male Wistar rats during 3 weeks. The first day of gavage was accompanied by a decrease in body weight and food intake in rats receiving *E.coli* which then gradually returned to similar to control levels (Fig. 4a,b). The refeeding period in the *E.coli* group, was however deficient, because no rebound of food intake necessary to regain lost body weight was observed. Efficiency of gavage with *E.coli* was confirmed by detection in rat faeces of abundant *E.coli K12* cDNA after gavage which was low prior to gavage (Fig. 4c). After gavage, plasma levels of anti-ClpB IgG were increased in rats that received *E.coli* as compared to controls (Fig. 4d) while concentrations of total IgG did not change, indicating a selective immune response to *E.coli* antigens. Plasma anti-α-MSH IgM (Fig. 4d), but not anti-α-MSH IgG were stimulated by *E.coli,* although increased anti-ACTH IgG were detected. Affinity kinetic analysis of α-MSH IgG (Fig. 4f-h) revealed their increased dissociation rates in rats receiving *E.coli.* These changes of α-MSH autoAbs may reflect a mild immune response to a novel antigen. In fact, low *E.coli* K12 cDNA levels in rat faeces before gavage indicates that this bacterial species was not a major gut commensal in studied rats. Different α-MSH autoAbs response found in *E.coli* gavaged rats vs. ClpB-immunized mice can also be related to both the route of administration and the dose of α-MSH-like antigens. Moreover, because *E.coli* K12 contain several α-MSH-like antigens, the cumulative immune response might depend on the novelty and antigenicity of each of them. Importantly, this animal model confirmed that changes in gut content of ClpB protein-expressing bacteria such as *E.coli,* is physiologically sensed by the host immune system producing anti-ClpB IgG and autoAbs cross-reactive with α-MSH. Therefore, detection of anti-ClpB autoAbs can be used as a biomarker of ClpB-expressing bacteria which would explain presence of anti-α-MSH autoAbs.

### Anti-ClpB antibodies in humans

While in controls ClpB IgG correlated negatively with the normal range of several psychological traits, in AN patients ClpB IgG correlated positively with the core psychopathological traits such as body dissatisfaction and drive for thinness (Table 1). In AN and BED patients psychopathological traits correlated oppositely with ClpB IgM autoAbs (Table 1). These correlations may signify that AN patients had been chronically exposed to some ClpB-containing bacteria, but BED patients, instead, had a recent infection with such bacteria.

**Table 1. Significant correlations between plasma levels of anti-ClpB IgG and IgM and psychological traits in eating disorder patients and controls (Contr.) assayed by the Eating Disorder Inventory-2. All Spearman's r *p<0.05, **p<0.01, except Pearson's r* p<0.05 for perfectionism. (n=65 Contr., n=27 AN, and n=14 BED).**

| | | | | |
|---|---|---|---|---|
| ClpB IgG (Contr.) | Maturity fears r= -0.31 * | Impulse regulation r= -0.26 * | Social insecurity r= -0.26 * | |
| ClpB IgG (AN) | Body dissatisfaction r= 0.4 * | Drive for thinness r= 0.35 * | Perfectionism r= 0.38 * | |
| ClpB IgM (AN) | Ineffectiveness r= -0.42 * | Interpersonal distrust r= -0.58 ** | Social insecurity r= -0.52 ** | Anhedonia r= -0.35 * |
| ClpB IgM (BED) | Bulimia r= 0.53 * | Perfectionism r= 0.6 * | Age r= -0.74 ** | |

### Discussion

These data revealed a molecular link between *E.coli* K12 bacteria expressing ClpB and the host melanocortin system via molecular mimicry between ClpB and α-MSH, implicating commensal gut bacteria in the host control of energy metabolism, motivated behaviour and emotion. The molecular mimicry concept was initially validated to explain development of autoimmune diseases caused mainly by viral proteins (Oldstone, M. B. Faseb J 1998, 12, 1255-1265). This work extends this concept by implicating it in the physiological interactions between commensal gut bacteria and host peptidergic signalling. It is, however, likely, that alterations of this physiological mechanism may lead to behavioural abnormalities resulting from either insufficient or excessive bacterial presence of peptide hormone mimetic proteins and corresponding changes in peptide hormone-reactive autoAbs production. Although direct binding of such mimetic proteins on peptide hormone receptors may influence peptidergic signaling, production of autoAbs cross-reactive with the host peptide hormones appears as a long-term regulatory mechanisms contributing to the host phenotype (Takagi, K. et al. Nat Commun in press, 2013). Significant correlations between anti-ClpB Abs and behavioural traits in healthy humans and in patients with AN and BED suggest that ClpB expressing bacteria may be relevant to the establishment of some normal or altered behavioural and emotional traits and, hence, may represent a new therapeutic target. Recent identification of ClpB inhibitors as potential antimicrobials (Martin, I. et al. Screening and Evaluation of Small Organic Molecules as ClpBInhibitors and Potential Antimicrobials. Journal of Medicinal Chemistry 2013, 56, 7177-7189). may help to clarify the relevance of ClpB containing bacteria to the host regulation of feeding and emotion.

Here the inventors identified and validated one bacterial protein, *E.coli* K12 ClpB as an α-MSH mimetic. ClpB has been well characterized for its functional role in bacteria (DeSantis, M. E. and Shorter, J. Molecular Cell Research 2012, 1823, 29-39) and, it is peculiar to find molecular mimicry between ClpB which is involved in the stress response in bacteria as a heat-shock protein and α-MSH which is a stress-related hormone and is also a potent anti-pyretic (Motohashi, K. et al. Proc Natl Acad Sci USA 1999, 96, 7184-7189, Charmandari, E. et al., Sci. Signal 2012, 5; Murphy, M. T. et al., Science 1983, 221, 192-193). Although such functional similarity might be coincidental, it may also suggest a phylogenetic link in development of coordinated responses to thermal stress in bacteria and host. Because the ClpB protein is present in several bacterial species they all should be able to stimulate α-MSH autoAbs.

## Claims

1. A composition comprising a bacterial ClpB protein, for use as a vaccine or as an immunogenic composition, for the immunization against a disease or disorder related to reduced appetite and/or anxiety; wherein said disease or disorder related to reduced appetite is selected from the group consisting of anorexia nervosa (AN), bulimia nervosa (BN), cachexia and wasting diseases; and wherein the bacterial ClpB protein comprises an amino acid sequence at least 80% identical to the amino acid sequence of SEQ ID NO: 1.

2. The composition for use according to claim **1,** for the treatment and/or prevention of a disease or disorder related to reduced appetite and/or anxiety; wherein said disease or disorder related to reduced appetite is selected from the group consisting of anorexia nervosa (AN), bulimia nervosa (BN), cachexia and wasting diseases.

3. The composition for use according to claim **1** or **2,** wherein the disease or disorder related to anxiety is selected from the group consisting of acute stress disorder, agoraphobia (with or without a history of panic disorder), generalized anxiety disorder (GAD), obsessive-compulsive disorder (OCD), panic disorder (with or without agoraphobia), phobias (including social phobia), posttraumatic stress disorder (PTSD), and anxiety associated with anorexia nervosa, bulimia nervosa, cachexia, or wasting disease.

4. The composition for use according to any one of claims **1** to **3,** wherein the bacterial ClpB protein comprises an amino acid sequence is 90 to 100% identical to the amino acid sequence of SEQ ID NO: 1.

5. The composition for use according to any one of claims **1** to **4,** wherein the bacterial ClpB protein is expressed by a bacterium of the genus *Enterobacteriaceae.*

6. The composition for use according to claim **5,** wherein the bacterium of the genus *Enterobacteriaceae* is *Escherichia coli.*

7. A non-therapeutic method of increasing appetite in a subject, comprising administering to said subject an effective amount of a composition comprising an anti-ClpB antibody.

8. The non-therapeutic method of increasing appetite according to claim **7,** wherein the anti-ClpB antibody is directed against both ClpB and α-melanocyte-stimulating hormone (α-MSH).

9. The non-therapeutic method of increasing appetite according to claim **7** or **8,** wherein the anti-ClpB antibody is directed against a ClpB protein comprising an amino acid sequence at least 80% identical to the amino acid sequence of SEQ ID NO: 1.

## Patentansprüche

1. Zusammensetzung, die ein bakterielles ClpB-Protein umfasst, zur Verwendung als ein Impfstoff oder als eine immunogene Zusammensetzung zur Immunisierung gegen eine Erkrankung oder Störung, die mit vermindertem Appetit und/oder Angstzuständen verbunden ist; wobei die Erkrankung oder Störung, die mit vermindertem Appetit verbunden ist, ausgewählt ist aus der Gruppe bestehend aus Anorexia nervosa (AN), Bulimia nervosa (BN), Kachexie und zehrenden Erkrankungen; und wobei das bakterielle ClpB-Protein eine Aminosäuresequenz umfasst, die zu mindestens 80 % mit der Aminosäuresequenz aus SEQ ID-Nr.: 1 identisch ist.

2. Zusammensetzung zur Verwendung nach Anspruch **1** zur Behandlung und/oder Vorbeugung einer Erkrankung oder Störung, die mit vermindertem Appetit und/oder Angstzuständen verbunden ist; wobei die Erkrankung oder Störung, die mit vermindertem Appetit verbunden ist, ausgewählt ist aus der Gruppe bestehend aus Anorexia nervosa (AN), Bulimia nervosa (BN), Kachexie und zehrenden Erkrankungen.

3. Zusammensetzung zur Verwendung nach Anspruch **1** oder **2,** wobei die Erkrankung oder Störung, die mit Angstzuständen verbunden ist, ausgewählt ist aus der Gruppe bestehend aus akuter Belastungsstörung, Agoraphobie (mit oder ohne einer Vorgeschichte von Panikstörung), generalisierter Angststörung (GAD), Zwangsstörung (OCD), Panikstörung (mit oder ohne Agoraphobie), Phobien (einschließlich sozialer Phobie), posttraumatischer Belastungsstörung (PTSD) und Angstzuständen, die mit Anorexia nervosa, Bulimia nervosa, Kachexie oder zehrender Erkrankung assoziiert sind.

4. Zusammensetzung zur Verwendung nach einem der Ansprüche **1** bis **3,** wobei das bakterielle ClpB-Protein eine Aminosäuresequenz umfasst, die zu 90 bis 100 % mit der Aminosäuresequenz aus SEQ ID-Nr.: 1 identisch ist.

5. Zusammensetzung zur Verwendung nach einem der Ansprüche **1** bis **4,** wobei das bakterielle ClpB-Protein von einem Bakterium der Gattung *Enterobacteriaceae* exprimiert wird.

6. Zusammensetzung zur Verwendung nach Anspruch **5,** wobei es sich bei dem Bakterium der Gattung *Enterobacteriaceae* um *Escherichia coli* handelt.

7. Nicht therapeutisches Verfahren zur Appetitsteigerung bei einem Subjekt, das das Verabreichen einer wirksamen Menge einer einen Antikörper gegen ClpB umfassenden Zusammensetzung an das Subjekt umfasst.

8. Nicht therapeutisches Verfahren zur Appetitsteigerung nach Anspruch **7,** wobei der Antikörper gegen ClpB gegen sowohl ClpB als auch α-Melanozyten stimulierendes Hormon (α-MSH) gerichtet ist.

9. Nicht therapeutisches Verfahren zur Appetitsteigerung nach Anspruch **7** oder **8,** wobei der Antikörper gegen ClpB gegen ein ClpB-Protein gerichtet ist, das eine Aminosäuresequenz umfasst, die zu mindestens 80 % mit der Aminosäuresequenz aus SEQ ID-Nr.: 1 identisch ist.

## Revendications

1. Composition comprenant une protéine bactérienne ClpB, pour son utilisation comme vaccin ou comme composition immunogène, pour l'immunisation contre une maladie ou un trouble lié à une diminution de l'appétit et / ou à l'anxiété; dans laquelle la maladie ou le trouble lié à un appétit réduit est choisi dans le groupe constitué de l'anorexie mentale (AM), de la boulimie mentale (BM), de la cachexie et des maladies de dépérissement; et dans laquelle la protéine bactérienne ClpB comprend une séquence d'acides aminés au moins 80% identique à la séquence d'acides aminés de SEQ ID NO: 1.

2. Composition pour son utilisation selon la revendication **1,** pour le traitement et / ou la prévention d'une maladie ou d'un trouble lié à une diminution de l'appétit et / ou à l'anxiété ; dans laquelle ladite maladie ou trouble lié à l'appétit réduit est choisi dans le groupe constitué de l'anorexie mentale (AM), de la boulimie mentale (BM), de la cachexie et des maladies de dépérissement.

3. Composition pour son utilisation selon la revendication **1** ou **2,** dans laquelle la maladie ou le trouble lié à l'anxiété est choisi dans le groupe comprenant le trouble de stress aigu, l'agoraphobie (avec ou sans antécédents de trouble panique), le trouble anxieux généralisée (TAG), le trouble obsessionnel-compulsif (TOC), le trouble panique (avec ou sans agoraphobie), les phobies (y compris la phobie sociale), le trouble de stress post-traumatique (TSPT) et l'anxiété associée à l'anorexie mentale, à la boulimie mentale, à la cachexie ou aux maladies de dépérissement.

4. Composition pour son utilisation selon l'une quelconque des revendications **1** à **3,** dans laquelle la protéine bactérienne ClpB comprend une séquence d'acides aminés identique de 90 à 100% à la séquence d'acides aminés de SEQ ID NO: 1.

5. Composition pour son utilisation selon l'une quelconque des revendications **1** à **4,** dans laquelle la protéine bactérienne ClpB est exprimée par une bactérie du genre *Enterobacteriaceae.*

6. Composition pour son utilisation selon la revendication **5,** dans laquelle la bactérie du genre *Enterobacteriaceae* est *Escherichia coli.*

7. Méthode non thérapeutique pour augmenter l'appétit chez un sujet, comprenant l'administration audit sujet d'une quantité efficace d'une composition comprenant un anticorps anti-ClpB.

8. La méthode non thérapeutique pour augmenter l'appétit selon la revendication **7,** dans laquelle l'anticorps anti-ClpB est dirigé contre la ClpB et contre l'hormone stimulant les α-mélanocytes (a-MSH).

9. La méthode non thérapeutique d'augmentation de l'appétit selon la revendication **7** ou **8,** dans laquelle l'anticorps anti-ClpB est dirigé contre une protéine ClpB comprenant une séquence d'acides aminés au moins 80% identique à la séquence d'acides aminés de SEQ ID NO: 1.
